(19)

Europäisches
Patentamt
European
Patent Office
Office européen
des brevets

(11) **EP 2 649 942 A1**

(12) **EUROPEAN PATENT APPLICATION**

(43) Date of publication:
**16.10.2013 Bulletin 2013/42**

(51) Int Cl.:
***A61B 6/04*** (2006.01)    ***A61B 6/00*** (2006.01)

(21) Application number: **13162335.7**

(22) Date of filing: **04.04.2013**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br><br>(30) Priority: **11.04.2012  JP 2012090497**<br>       **14.03.2013  JP 2013052240**<br><br>(71) Applicant: **Fujifilm Corporation**<br>**Minato-ku**<br>**Tokyo (JP)** | (72) Inventors:<br> • **Otokuni, Shinji**<br>   **Kanagawa (JP)**<br> • **Inoue, Tomoki**<br>   **Kanagawa (JP)**<br><br>(74) Representative: **Klunker . Schmitt-Nilson . Hirsch**<br>**Patentanwälte**<br>**Destouchesstrasse 68**<br>**80796 München (DE)** |

(54) **Radiographic imaging device, radiographic imaging method and program storage medium**

(57)    A radiographic imaging device includes a compression plate, irradiation detection sections, and a correction section. The compression plate that compresses an imaging site of a subject between the compression plate and an imaging face of an imaging table, and that inclines with respect to the imaging face in accordance with the imaging site during the compression. The irradiation detection sections, each of which is provided at a different position on the imaging face and detects an irradiation amount of irradiated radiation. The correction section that corrects detection results from the plural irradiation detection sections using correction coefficients that are based on the thickness of the imaging site compressed by the compression plate and on the positions of the plural irradiation detection sections.

FIG.8

EP 2 649 942 A1

**Description**

BACKGROUND OF THE INVENTION

Technical Field

The present invention relates to a radiographic imaging device, a radiographic imaging method, and a program storage medium, and in particular relates to a radiographic imaging device, a radiographic imaging method, and a program storage medium that performs imaging of a radiographic image on an imaging site of a subject with the imaging site being compressed.

Related Art

Radiographic imaging devices that perform radiographic imaging for the purpose of medical diagnoses are known. An example of an application of this type of radiographic imaging device is mammography in which a breast of a subject is imaged for the purpose of early detection of breast cancer.
In such radiographic imaging devices, radiographic imaging is generally performed by detecting radiation that has passed through the imaging site (a breast in the case of mammography) with the imaging site of the subject being compressed by, for example, a compression plate. Compressing and holding the imaging site in this way makes it possible to prevent blurring of the radiographic image, as well as to reduce the radiation amount since the imaging site becomes thinner.
In such radiographic imaging devices, detection sensors that detect a radiation amount are generally provided. The imaging site is irradiated with radiation prior to imaging, and imaging conditions are set based on detection results of the transmitted radiation (radiation amount) detected by the detection sensors.
Japanese National-Phase Publication No. 2005-509482 discloses a breast X-ray imaging device provided with AEC detectors. This document discloses a technology that corrects and adjusts imaging parameter values (in particular exposure duration) based on the thickness of a breast along a beam direction. Japanese Patent Application Laid-Open (JP-A) No. 2008-86383 discloses a technology in which imaging conditions are set by finding out the amount of radiation transmitted through a breast based on the thickness of the breast and radiation measurement results of an AEC sensor.
Such related technology sets imaging conditions under the assumption that the thickness of a breast compressed by a compression plate is even.
However, in cases in which a breast does not have a uniform thickness and the thickness of the breast varies depending on the positions where detection sensors are provided, radiation is less readily transmitted through the breast at areas with greater thickness, and is more readily transmitted at thinner areas. Accordingly, the precision of detecting the transmitted radiation may be lowered when such related technology is applied in this case. Moreover, when the transmitted radiation amount is relatively small, it may be difficult to determine whether the small amount of radiation is due to the thick breast, or due to the presence of tissue through which radiation is not readily transmitted.

SUMMARY

The present invention is directed towards providing a radiographic imaging device, a radiographic imaging method, and a radiographic imaging program storage medium capable of improving the detection precision of a transmitted radiation amount.
A first aspect of the present invention is a radiographic imaging device including: a compression plate that compresses an imaging site of a subject between the compression plate and an imaging face of an imaging table, and that inclines with respect to the imaging face in accordance with the imaging site during the compression; plural irradiation detection sections, each of which is provided at a different position on the imaging face and detects an irradiation amount of irradiated radiation; and a correction section that corrects detection results from the plural irradiation detection sections using correction coefficients that are based on the thickness of the imaging site compressed by the compression plate and on the positions of the plural irradiation detection sections.
The above aspect further include an estimation section that estimates the thickness of the imaging site compressed by the compression plate for each of the positions of the plural irradiation detection sections, wherein the correction section determines a correction coefficient for correcting each of the detection results from the plural irradiation detection sections based on the thickness of the imaging site estimated by the estimation section, and corrects each of the detection results from the plural irradiation detection sections using the determined correction coefficient.
In the above aspect, the estimation section may detect a distance between the compression plate and the imaging face, and estimates the thickness of the imaging site according to the detected distance.
The above aspect may further include an inclination detection section that detects the inclination of the compression plate with respect to the imaging face, wherein the estimation section further estimates the thickness of the imaging site based on the inclination of the compression plate detected by the inclination detection section.

In the above aspect, the plural irradiation detection sections may be provided along an inclination direction of the compression plate.

The above aspect may further include a radiation detector including plural pixels that accumulate charges in response to radiation and are disposed in a two-dimensional formation in a pixel region facing the imaging face; a region determination section that determines a plural regions in the pixel region based on a distance between the compression plate and the imaging face and on the inclination of the compression plate; and an irradiation detection pixel determination section that determines, from among the plural pixels, irradiation detection pixels that are to be used as the irradiation detection sections, such that the irradiation detection pixels are disposed at a specific pixel density in each of the plural regions, wherein the correction coefficient is predetermined for each of the plural regions, and the correction section corrects the detection results of the irradiation detection pixels for each of the regions using the predetermined correction coefficient.

In the above aspect, the specific pixel density may increase as the distance between the compression plate and the imaging face increase.

In the above aspect, the irradiation detection pixel determination section may determine, as the irradiation detection pixels for each of the plural regions, one pixel from each of a predetermined number of pixels arrayed along an inclination direction of the compression plate, the predetermined number being determined for each of the regions.

In the above aspect, the irradiation detection pixel determination section may determine, as the irradiation detection pixels for each of specific positions in the plural regions, a predetermined number of pixels, the predetermined number being determined for each of the regions; and the correction section may correct an average value of detection results of the irradiation detection pixels for each of the specific positions using the correction coefficient.

In the above aspect, the irradiation detection pixel determination section may determine, as the irradiation detection pixels for each of the specific positions, the predetermined number of pixels, which are arrayed along a direction different from the inclination direction.

In the above aspect, the irradiation detection pixel determination section may determine, as the irradiation detection pixels, the predetermined number of pixels, which are arranged in a two-dimensional formation centering on the specific positions.

In the above aspect, the irradiation detection pixel determination section may identify a position of the imaging site on the imaging face based on a radiographic image in which the imaging site is imaged by the radiation detector, and determine the irradiation detection pixels from the plural pixels provided at positions corresponding to the identified position of the imaging site.

The above aspect may further include a radiation detector including plural pixels disposed in a two-dimensional formation in a pixel region facing the imaging face, the plural pixels being used for imaging of a radiographic image by accumulating charges in response to radiation and being used as irradiation detection pixels; a region determination section that determines plural regions in the pixel region based on a distance between the compression plate and the imaging face and on the inclination of the compression plate; and a detection section determination section that determines, for each of the plural regions, plural detection sections including a predetermined number of pixels, the predetermined number being determined for each of the regions and increasing as the thickness of the imaging site increases, wherein the correction coefficient is predetermined for each of the plural regions, and the correction section corrects average values of the detection results of the detection sections for each of the regions using the predetermined correction coefficient.

The above aspect may further include an adjustment section that adjusts at least one of a radiation amount or radiation characteristics of radiation for imaging a radiographic image of the imaging site, based on the detection results from the plural irradiation detection sections that have been corrected by the correction section.

In the above aspect, an object of adjustment by the adjustment section may be at least one object selected from the group consisting of a tube voltage of a radiation source that generates the radiation, a tube current of the radiation source, an irradiation duration of the radiation source, a type of target for generating bremsstrahlung radiation that is provided at the irradiation source, and a type of filter provided between the radiation source and the subject.

The above aspect may further include an image correction section that corrects an imaged radiographic image using the correction coefficient determined by the correction section.

The above aspect may further include a storage section that stores the correction coefficient.

Another aspect of the present invention is a radiographic imaging method in a radiographic imaging device including: compressing an imaging site of a subject between a compression plate and an imaging face of an imaging table while inclining the compression plate with respect to the imaging face in accordance with the imaging site; detecting an irradiation amount of irradiated radiation using plural irradiation detection sections, each of which is provided at a different position on the imaging face; and correcting detection results from the plural irradiation detection sections using correction coefficients that are based on the thickness of the imaging site compressed by the compression plate and on the positions of the plural irradiation detection sections.

The another aspect may further include adjusting at least one of a radiation amount or radiation characteristics of radiation with which the examination subject is irradiated, based on the corrected detection results of the plural irradiation detection sections.

An yet another aspect of the present invention is a computer readable storage medium stored with a program that causes a computer to execute radiographic imaging processing, the radiographic imaging processing including: compressing an imaging site of a subject between a compression plate and an imaging face of an imaging table while inclining the compression plate with respect to the imaging face in accordance with the imaging site; detecting an irradiation amount of irradiated radiation using plural irradiation detection sections, each of which is provided at a different position on the imaging face; and correcting detection results from the plural irradiation detection sections using correction coefficients that are based on the thickness of the imaging site compressed by the compression plate and on the positions of the plural irradiation detection.

According to the above aspects, it is possible to improve the detection precision of a transmitted radiation amount.

BRIEF DESCRIPTION OF THE DRAWINGS

Exemplary embodiments of the present invention will be described in detail based on the following figures, wherein:

Fig. 1 is a side view illustrating an example of a configuration of a radiographic imaging device of a first exemplary embodiment;

Fig. 2 is a configuration diagram illustrating an example of a configuration of a radiation source of a radiation irradiation section of the first exemplary embodiment;

Fig. 3 is a schematic configuration diagram illustrating an example of a radiation detector of the first exemplary embodiment;

Fig. 4 is a schematic diagram illustrating an example of the arrangement of AEC sensors in the first exemplary embodiment;

Fig. 5 is a function block diagram illustrating an example of a configuration of a radiographic imaging device of the first exemplary embodiment;

Fig. 6 is a flow chart illustrating an example of a flow of radiographic imaging processing in the first exemplary embodiment;

Fig. 7 is a flow chart illustrating an example of a flow of imaging condition setting processing in the first exemplary embodiment;

Fig. 8 is a schematic diagram illustrating a compressed state of a breast by a compression plate in the radiographic imaging device of the first exemplary embodiment;

Fig. 9 is a schematic diagram illustrating a relationship between the compression plate and an imaging table (imaging face) illustrated in Fig. 8;

Fig. 10 is a flow chart illustrating an example of a flow of radiographic image correction processing employing a correction coefficient in the radiographic imaging device of the first exemplary embodiment;

Fig. 11 is a schematic diagram illustrating an example of sensors provided in order to detect a separation distance between the compression plate and the imaging face;

Fig. 12 is a function block diagram illustrating a configuration of a radiographic imaging device of Example 1 of a second exemplary embodiment;

Fig. 13 is a flow chart illustrating a flow of imaging condition setting processing in the example 1 of the second exemplary embodiment;

Fig. 14 is a schematic diagram illustrating the arrangement of detection sensors and AEC pixels in the Example 1 of the second exemplary embodiment;

Fig. 15 is a flow chart illustrating a flow of radiographic image correction processing employing a correction coefficient in the radiographic imaging device of the Example 1 of the second exemplary embodiment;

Fig. 16 is a flow chart illustrating a flow of imaging condition setting processing in Example 2 of the second exemplary embodiment;

Fig. 17 is a schematic diagram illustrating the arrangement of detection sensors and AEC pixels in the Example 2 of the second exemplary embodiment;

Fig. 18 is a flow chart illustrating a flow of imaging condition setting processing in Example 3 of the second exemplary embodiment;

Fig. 19 is a schematic diagram illustrating the arrangement of detection sensors and AEC pixels in the Example 3 of the second exemplary embodiment;

Fig. 20 is a flow chart illustrating a flow of imaging condition setting processing in Example 4 of the second exemplary embodiment;

Fig. 21 is a schematic diagram illustrating the arrangement of detection sensors and AEC pixels in the Example 4 of the second exemplary embodiment;

Fig. 22 is a function block diagram illustrating a configuration of a radiographic imaging device of a third exemplary embodiment;

Fig. 23 is a flow chart illustrating a flow of imaging condition setting processing in the third exemplary embodiment; and

Fig. 24 is a schematic diagram illustrating the arrangement of detection sensors and AEC pixels in the third exemplary embodiment.

## DETAILED DESCRIPTION

First Exemplary Embodiment

Explanation is firstly given regarding a case in which AEC sensors are employed as irradiation amount detection sensors.

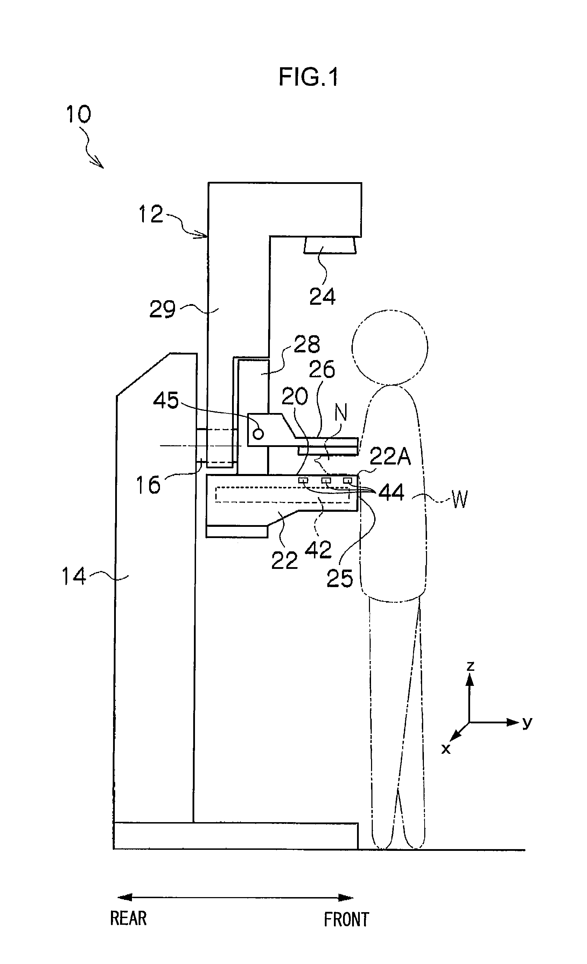

As shown in Fig. 1, a radiographic imaging device 10 of the present exemplary embodiment, which is referred to as mammography, for example, is a device that uses radiation (for example, X-rays) to image a breast N of a subject W with the subject W in an upright standing state. Note that in the following explanation, the side at the front nearest to the subject W in a state in which the subject W faces the radiographic imaging device 10 during imaging is treated as the device front side of the radiographic imaging device 10, and the far side further away from the subject W in this state is treated as the device rear side of the radiographic imaging device 10 (the y-axis directions in Fig. 1). Further, the left-right direction of the subject W in a state in which the subject W faces the radiographic imaging device 10 is treated as the device left-right direction of the radiographic imaging device 10 (the x-axis directions in Fig. 1).

The radiographic imaging device 10 of the present exemplary embodiment may also be a device that images the breast N of the subject W in a seated state, for example with the subject W sitting in a chair (including in a wheelchair), and may be a device capable of imaging the left and right breasts N of the subject W individually in a state in which the upper body of the subject W is in an upright state.

As shown in Fig. 1, the radiographic imaging device 10 includes a measurement section 12 that is substantially C-shaped in side view and provided at the device front side, and a base section 14 that supports the measurement section 12 from the device rear side.

The measurement section 12 includes: an imaging table 22 formed with a flat, plane-shaped imaging face 20 that contacts the breast N of the subject W who is in an upright state; a compression plate 26 that compresses the breast N between the compression plate 26 and the imaging face 20 of the imaging table 22; and a holder section 28 that supports the imaging table 22 and the compression plate 26. The measurement section 12 further includes a radiation irradiation section 24 that irradiates radiation for examination towards the imaging face 20, and a support section 29 that is separated from the holder section 28 and supports the radiation irradiation section 24.

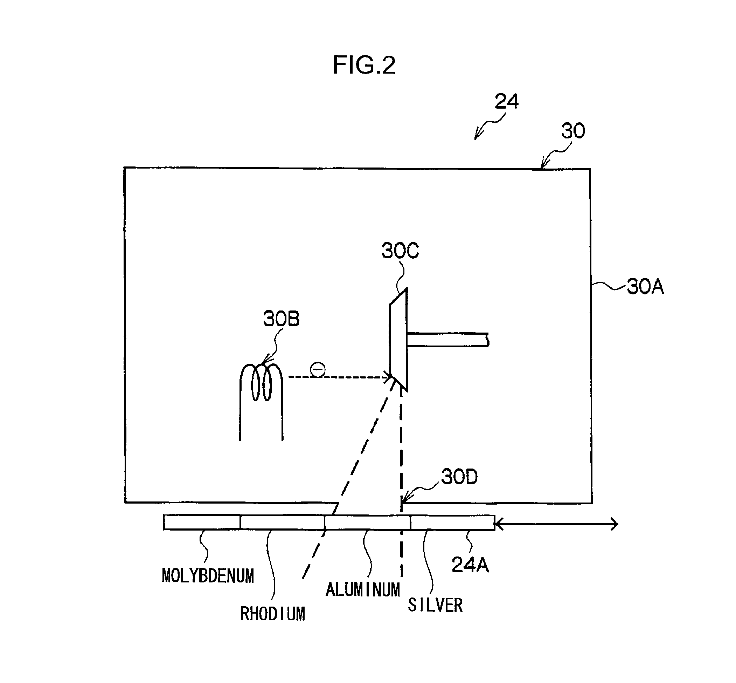

The radiation irradiation section 24 includes a radiation source 30 such as a tube, and a filter 24A. Fig. 2 illustrates a configuration of the radiation irradiation section 24 according to the present exemplary embodiment.

The radiation source 30 is provided with a cathode 30B configured including a filament, and a target (anode) 30C inside a housing 30A. Thermions emitted from the cathode 30B are accelerated and focused by the potential difference between the cathode and the anode, and collide with the target 30C, thereby generating bremsstrahlung. In the present exemplary embodiment, plural radiation sources 30 are provided, and different types of metal are employed for the targets 30C, such as tungsten, molybdenum and rhodium. The strength of the generated bremsstrahlung varies depending on the type of the target.

The radiation generated by the radiation source 30 is externally irradiated through a window 30D provided in the housing 30A. The window 30D portions of the radiation irradiation section 24 are provided with the filters 24A that are respectively formed from films of molybdenum, rhodium, aluminum, and silver.

The filters 24A can be moved or replaced in the radiation irradiation section 24 of the present exemplary embodiment using a mechanical mechanism (for example, guide rails not shown in the drawings). The characteristics of the radiation irradiated from the radiation irradiation section 24 change when the filters 24A are changed.

The measurement section 12 is further provided with a rotation shaft 16 that is rotatably supported on the base section 14. The rotation shaft 16 is fixed to the support section 29, such that the rotation shaft 16 and the support section 29 rotates as one body.

The holder section 28 can be switched between a coupled state in which it rotates with the rotation shaft 16 combined as one body, and a free state in which it rotates separated from the rotation shaft 16. Specifically, gears are respectively provided to the rotation shaft 16 and the holder section 28, in order to switch between a meshed state and an unmeshed state between these gears. Various mechanical components may be employed for switching between transmission and non-transmission of the rotation force of the rotation shaft 16.

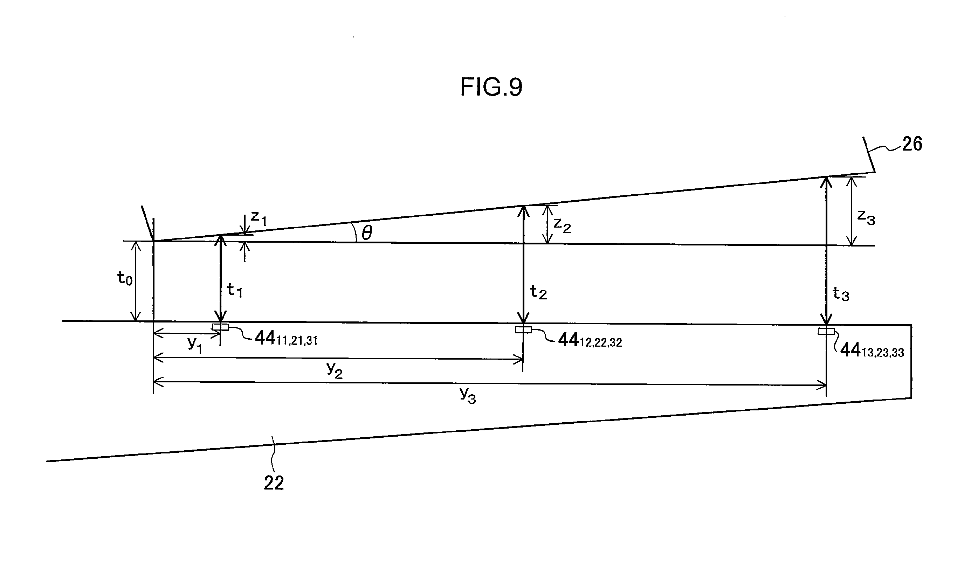

The holder section 28 supports the imaging table 22 and the radiation irradiation section 24 such that the imaging face 20 is separated from the radiation irradiation section 24 by a specific distance. The holder section 28 retains the compression plate 26 such that the compression plate 26 can be moved by sliding, so that it is possible to vary the distance between the compression plate 26 and the imaging face 20. The holder section 28 outputs the slide position of the compression plate 26 (for example, the distance between the imaging face 20 and the compression plate 26, see slide position $z_0$ in Fig. 9).

The compression plate 26 compresses the breast N between the compression plate 26 and the imaging face 20 of the imaging table 22, and a member that transmits radiation is employed for the compression plate 26. The compression plate 26 of the present exemplary embodiment is configured so as to perform inclined compression by inclining to follow the breast N when compressing the breast N against the imaging table 22 (detailed explanation to follow). The compression plate 26 has an angle sensor 45 that detects and outputs the angle of inclination of the compression plate 26 when the breast N is compressed (the angle of inclination from the holder section 28 towards a chest wall 25 in Fig. 1, see the angle of inclination θ in Fig. 9).

From the perspective of radiation transmissivity and strength, the imaging face 20 that contacts the breast N is formed for example from carbon. A radiation detector 42 that detects radiation is disposed inside the imaging table 22. The radiation detector 42 receives irradiated radiation that has passed through the breast N and the imaging face 20 and carries image data, stores the image data, and outputs the stored image data, and is configured as a Flat Panel Detector (FPD), for example, which converts radiation into digital data and outputs this digital data. The radiation detector 42 outputs image data expressing a radiographic image when irradiated with radiation. In the present exemplary embodiment, image data that expresses a radiographic image of the breast N is obtained by the radiation detector 42.

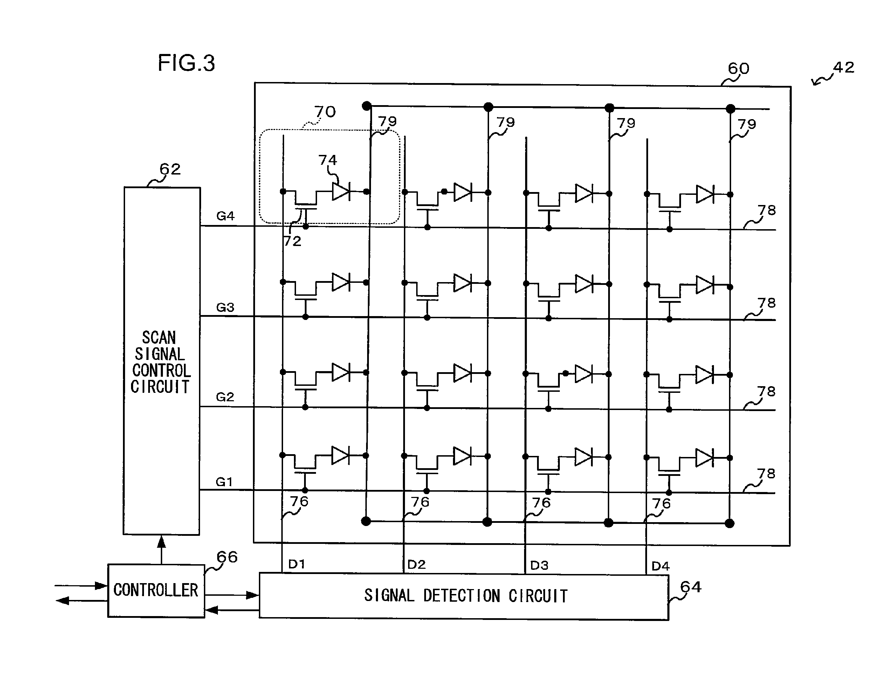

A specific example of the radiation detector 42 of the present exemplary embodiment is illustrated in the schematic configuration diagram of Fig. 3. The radiation detector 42 of the present exemplary embodiment illustrated in Fig. 3 employs an indirect-conversion radiation detection element 60 that firstly converts radiation into light, and then converts the light into charges. Note that a scintillator that converts radiation into light is omitted from illustration in Fig. 3. The radiation detection element employed in the radiation detector 42 is not limited thereto, and a direct-conversion radiation detection element that directly converts radiation to charges in a semiconductor layer and accumulates the charges may also be employed. In such cases, the direct-conversion radiation detection element generates charges upon irradiation with radiation.

The radiation detection element 60 includes plural pixels 70 disposed in a matrix formation. Each of the pixels 70 includes a sensor portion 74 that generates charges upon receipt of light and accumulates the generated charges, and a TFT switch 72 that is a switching element for reading the charges accumulated in the sensor portion 74. In the present exemplary embodiment, charges are generated in the sensor portion 74 when illuminated with light converted by the scintillator.

Plural pixels 70 are disposed in a matrix formation in one direction (the lateral direction in Fig. 3, also referred to below as the "row direction") and in a direction intersecting with the one direction (the vertical direction in Fig. 3, also referred to below as the "column direction"). Although Fig. 3 shows a simplified array of the pixels 70, there are, for example, 1024 × 1024 pixels 70 respectively disposed in the row direction and in the column direction.

In the radiation detection element 60, plural scan lines 78 serving as control lines for switching the TFT switches 72 ON/OFF, and plural signal lines 76 for reading the charges accumulated in the sensor portions 74, are disposed on a substrate (not shown in the drawings) so as to intersect with each other. In the present exemplary embodiment, one of the scan lines 78 is provided for each pixel row in the one direction, and one of the signal lines 76 is provided for each pixel column in the intersecting direction.

The radiation detection element 60 further has common electrode lines 79 that are parallel to each of the signal lines 76. One end and the other end of each of the common electrode lines 79 are connected in parallel, with the one end connected to a power source (not shown in the drawings) that supplies a predetermined bias voltage. The sensor portions 74 are connected to the corresponding common electrode lines 79, and a bias voltage is applied to the sensor portions 74 through the common electrode lines 79.

Scan signals for switching each of the TFT switches 72 flow in the scan lines 78. Accordingly, each of the TFT switches 72 is switched ON/OFF by the scan signals that flow through the scan lines 78. According to the switching state of the TFT switch 72 of each of the pixels 70, electrical signals corresponding to the charges accumulated in each of the pixels 70 flow in the signal lines 76. Specifically, an electrical signal corresponding to the accumulated charges flows in each of the signal lines 76 due to the on-state of TFT switch 72 of any of the pixels 70 connected to that signal line 76.

A signal detection circuit 64 that detects electrical signals flowing out from each of the signal lines 76 is connected to each of the signal lines 76. A scan signal control circuit 62 that outputs scan signals to switch ON/OFF for each of the TFT switches 72 on each of the scan lines 78 is connected to each of the scan lines 78. Although Fig. 3 shows a single signal detection circuit 64 and a single scan signal control circuit 62, there may be plural signal detection circuits 64 and plural scan signal control circuits 62, and a specific number of the signal lines 76 and the scan lines 78 may be respectively connected to each of the signal detection circuits 64 and the signal control circuits 62.

An amplification circuit (not shown in the drawings) that amplifies input electrical signals is built into the signal detection circuit 64 for each of the signal lines 76. In the signal detection circuit 64, electrical signals input from each of the signal lines 76 are amplified by the amplification circuit and converted into digital signals by an analogue-digital converter (ADC, not shown in the drawings).

A controller 66, which performs specific processing such as noise removal on the digital signals converted in the signal detection circuit 64, and which outputs control signals to the signal detection circuit 64 at a signal detection timing, is connected to the signal detection circuit 64. The controller 66 is also connected to the scan signal control circuit 62, and outputs to the scan signal control circuit 62 control signals expressing output timings of scan signals.

The controller 66 of the present exemplary embodiment is configured by a microcomputer and includes, for example, a Central Processing Unit (CPU), Read Only Memory (ROM), Random Access Memory (RAM), and a non-volatile storage section configured by a flash memory. The controller 66 generates and outputs an image expressing irradiated radiation based on the electrical signals expressing charges information of the pixels 70 input from the signal detection circuit 64.

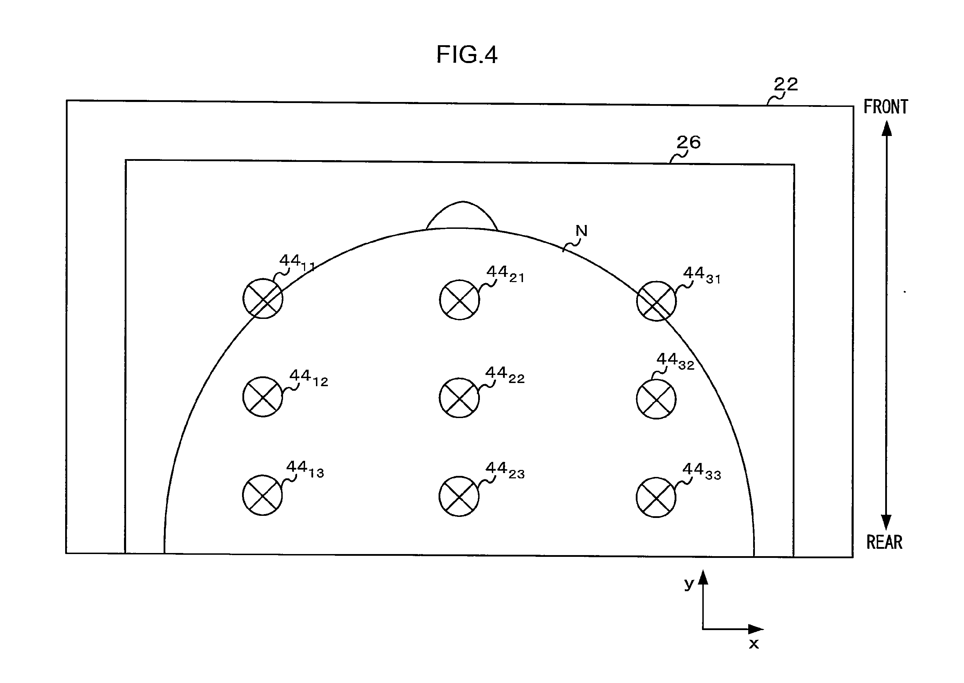

Plural Automatic Exposure Control (AEC) sensors 44 are provided inside the imaging table 22 together with the radiation detector 42. The AEC sensors 44 are radiation amount detection sensors that detect radiation amounts transmitted through the breast N during pre-irradiation performed prior to radiographic imaging and output the detection results. Fig. 4 illustrates an example of the arrangement of the AEC sensors 44 of the present exemplary embodiment. Fig. 4 is a schematic diagram illustrating a plan view seen from the upper side of the radiographic imaging device 10. The AEC sensors 44 are provided at different positions on the imaging face 20 of the imaging table 22. As shown in Fig. 4, by way of an example, $3 \times 3 = 9$ AEC sensors $44_{xy}$ (AEC sensors $44_{11}$ to $44_{33}$) are provided in the present exemplary embodiment. Note that in the following explanation, the plural AEC sensors are referred simply to as "AEC sensors 44" when they are not differentiated individually. In the present exemplary embodiment, the "radiation amount" refers to the product of a tube voltage (mA) when radiation is being output and an irradiation duration (sec), which is known as a mAs value.

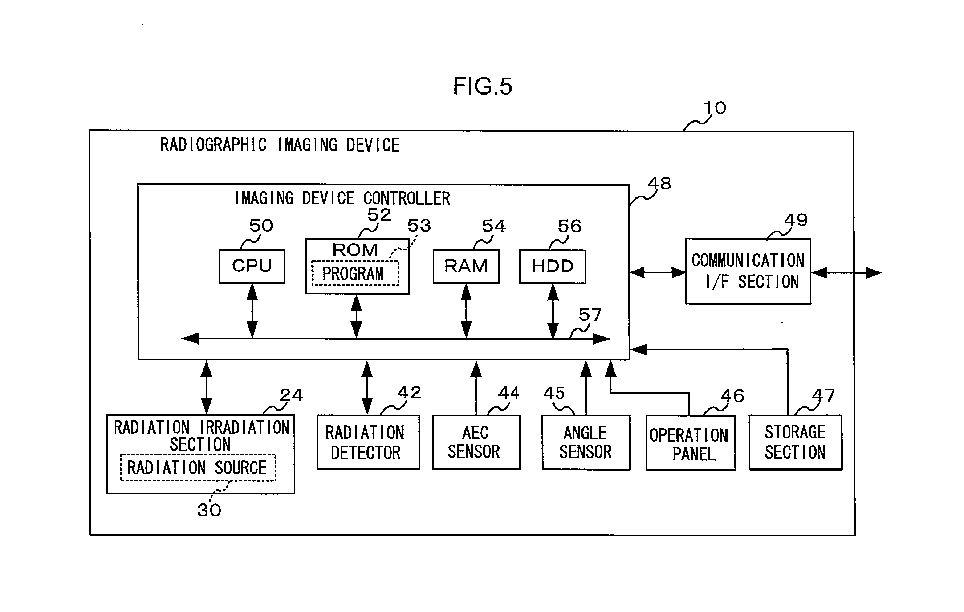

Fig. 5 is a function block diagram illustrating an example of a configuration of the radiographic imaging device 10 of the present exemplary embodiment. As shown in Fig. 5, the radiographic imaging device 10 includes the radiation irradiation section 24, the radiation detector 42, the AEC sensors 44, the angle sensor 45, an operation panel 46, a storage section 47, an imaging device controller 48, and a communication I/F section 49.

The imaging device controller 48 includes a Central Processing Unit (CPU) 50, Read Only Memory (ROM) 52, Random Access Memory (RAM) 54, and a Hard Disk Drive (HDD) 56. These sections are connected together by a bus 57, such as a control bus or a data bus, so as to be capable of transmitting and receiving data and the like between each other.

The CPU 50 performs control of the radiographic imaging device 10 overall, and more specifically, performs control by executing a program 53 stored in the ROM 52.

Although the present exemplary embodiment employs a configuration in which the program 53 is pre-installed, there is no limitation thereto. For example, the program 53 may be stored on a storage medium such as a CD-ROM or a removable disk and then may be installed in the ROM 52 from the storage medium, or the program 53 may be installed in the ROM 52 from an external device through a communication line such as the internet. The RAM 54 reserves a working space for use during execution of the program 53 by the CPU 50. The HDD 56 stores and holds various types of data.

The imaging device controller 48 is connected to the radiation irradiation section 24, the radiation detector 42, the AEC sensors 44, the angle sensor 45, the operation panel 46, the storage section 47, and the communication I/F section 49.

Upon receipt of an irradiation instruction from an operator (user) through the operation panel 46 (exposure switch), the imaging device controller 48 irradiates the imaging face 20 with radiation from the radiation source 30 provided in the radiation irradiation section 24, according to an imaging menu that has been set based on designated exposure conditions.

The operation panel 46 functions to set various kinds of operation data, such as exposure conditions and orientation data, and various kinds of operation instruction. The exposure conditions set via the operation panel 46 include information such as tube voltage, tube current, irradiation duration, and orientation data. The orientation data designated via the operation panel 46 includes information representing imaging positions (imaging orientation or angle) in cases in which imaging of the breast N is performed from plural directions. Various kinds of operation data such as the exposure conditions and the posture data and various kinds of operation instruction may be set by the user via the operation panel 46. Alternatively, these data may be obtained from another control device (such as a Radiology Information System: RIS, which is a system that manages data for medical examinations and diagnosis using radiation), or may be stored in advance in the HDD 56.

After various kinds of data are set by the operation panel 46, the imaging device controller 48 irradiates radiation from the radiation irradiation section 24 onto the imaging site (breast N) of the subject W according to an imaging menu that has been set based on the various kinds of data that has been set, and performs radiographic imaging. In cases in which imaging is performed from plural directions, the imaging device controller 48 adjusts the orientation of the holder section 28 to a state in which the imaging face 20 is facing upwards, and adjusts the orientation of the support section 29 to a state in which the radiation irradiation section 24 is positioned above the imaging face 20.

The storage section 47 stores a determined correction coefficient (explained in detail later). In the present exemplary embodiment, data such as y-axis direction positions y where the AEC sensors 44 are provided, and a correspondence relationship between a distance (separation) t and the correction coefficient (explained in detail later) are stored in advance in the storage section 47.

The communication I/F section 49 is a communication interface that functions to transmit radiographic images imaged by the radiographic imaging device 10, and to transmit and receive various kinds of data.

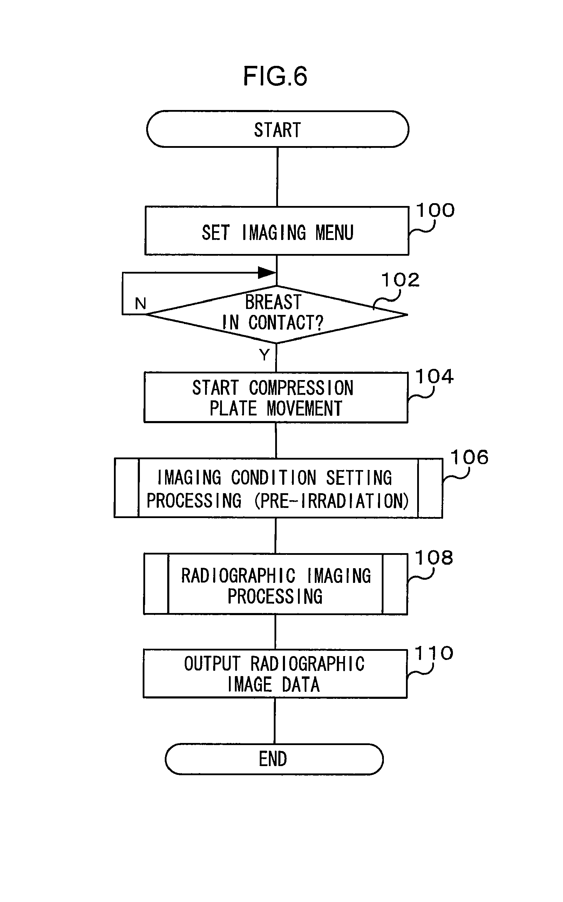

Next, explanation follows regarding a flow of processing in radiographic imaging by the radiographic imaging device 10 of the present exemplary embodiment. Fig. 6 is a flow chart illustrating a flow of radiographic imaging processing in the present exemplary embodiment.

In a case of performing radiographic imaging with the radiographic imaging device 10, firstly the imaging menu is set at step 100. After the imaging menu has been set, imaging is performed according to this imaging menu.

The subject W places her breast N on the imaging face 20 of the radiographic imaging device 10. An operator (user) performs positioning of the subject W within the field of irradiation of the radiation irradiation section 24 up to the chest wall 25 without putting unreasonable demands on the posture of the subject W. Then, at step 102, determination is made as to whether or not the breast N of the subject W has made contact with the imaging face 20, namely whether or not the positioning has been performed. If positioning has not yet been performed, negative determination is made and the radiographic imaging device 10 stands by. If positioning has been performed, positive determination is made and processing proceeds to step 104. In the present exemplary embodiment, it is determined that the positioning has been performed if a compression start operation is instructed by the operator via the operation panel 46.

At the next step 104, movement of the compression plate 26 towards the imaging face 20 is started. Positioning of the subject W is fixed by the breast N being compressed by the compression plate 26.

At the next step 106, imaging condition setting processing accompanying pre-irradiation is performed. As described in detail below, in the imaging condition setting processing, after movement of the compression plate 26 is stopped, a separation t between the compression plate 26 and the imaging face 20 is detected, the detection values of the AEC sensors 44 are corrected according to the detected separation t, and imaging conditions are then set using the corrected detection values.

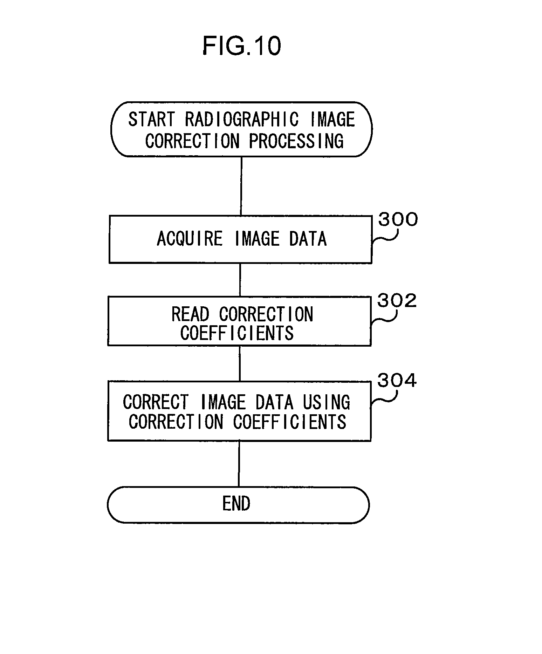

After the imaging conditions have been set, at the next step 108, radiographic imaging processing is performed under the set imaging conditions. Firstly, the radiation is irradiated from the radiation source 30 of the radiation irradiation section 24 onto the imaging face 20. The radiation irradiated from the radiation irradiation section 24 reaches the radiation detector 42 after passing through the breast N. The radiation detector 42 outputs image data to the imaging device controller 48 in response to irradiation of radiation. The imaging device controller 48 performs various processing, such as the correction using the correction coefficient (described later in reference to Fig. 10) or shading correction, on the image data expressing the radiographic image.

At the next step 110, the radiographic image expressing the corrected image data is output to an external device, and the radiographic imaging processing is ended.

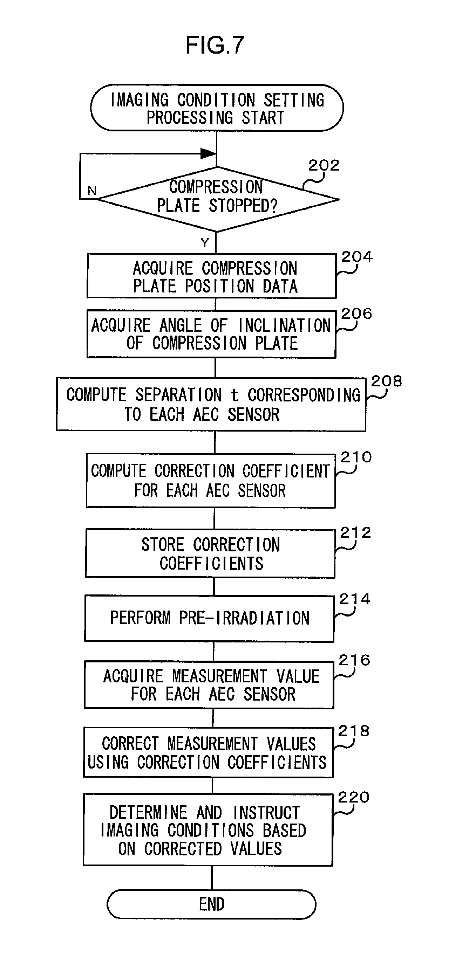

Detailed explanation follows regarding the imaging condition setting processing of step 106 in Fig. 6 described above. Fig. 7 is a flow chart illustrating an example of a flow of the imaging condition setting processing of the present exemplary embodiment.

At step 202, determination is made as to whether or not movement of the compression plate 26 has stopped. If the movement has not yet stopped, negative determination is made, and the radiographic imaging device 10 stands by. If the movement has stopped, positive determination is made and processing proceeds to step 204.

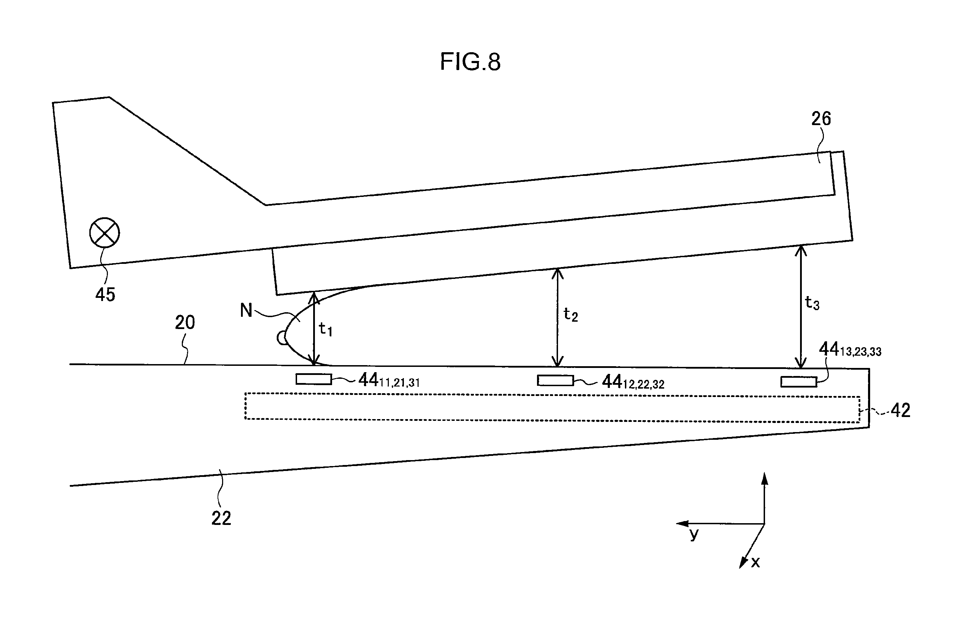

Fig. 8 is a schematic diagram illustrating a compressed state of the breast N by the compression plate 26. As shown in Fig. 8, in the present exemplary embodiment, the compression plate 26 performs inclined compression by following the shape of the breast N. Specifically, the breast N is compressed in a state in which the compression plate 26 is inclined so as to be higher at the chest wall side and lower at the leading end portion of the breast N.

It is necessary to compress the entire imaging site when the imaging site is compressed by the compression plate 26. For example, in a case such as the present exemplary embodiment in which the imaging site is the breast N, it is necessary to compress the breast N from the chest wall side to the leading end portion of the breast N. Thus, if the compression plate 26 compresses parallel (horizontal compression) with respect to the imaging table 22 (imaging face 20), heavy pressure is required for compression, which causes pain to the subject W due to squashing the breast N and due to stretching the skin in the vicinity of the chest wall. Therefore, in the present exemplary embodiment, the compression plate 26 performs inclined compression that follows the shape of the breast N as described above. Inclined compression such as in the present exemplary embodiment enables to alleviate the pain caused to the subject W by reducing the compression force compared to cases in which horizontal compression is performed and by reducing the stretching of the skin of the subject W.

As shown in Fig. 8, in the present exemplary embodiment, the breast N is compressed with the compression plate 26 in a tilted state from the holder section 28 towards the chest wall 25. The thickness of the breast N is accordingly greatest at the chest wall side, becomes gradually thinner on progression along the y-axis direction towards the leading end portion of the breast N. The thickness of the breast N does not significantly change along the x-axis direction. The thickness of the breast N at the positions where each of the AEC sensors $44_{11}$, $44_{21}$, $44_{31}$ are provided is therefore substantially the same as one another (the separation $t_1$ in Fig. 8). Similarly, the thickness of the breast N at the positions where each of the AEC sensors $44_{12}$, $44_{22}$, $44_{32}$ are provided is also substantially the same as one another (the separation $t_2$ in Fig. 8), and the thickness of the breast N at the positions where each of the AEC sensors $44_{13}$, $44_{23}$, $44_{33}$ are provided is also substantially the same as one another (the separation $t_3$ in Fig. 8), such that separation $t_3$ > separation $t_2$ > separation $t_1$. In the present exemplary embodiment, the thickness of the breast N is estimated from the separation t between the compression plate 26 and the imaging face 20.

After the breast N has been compressed in this way, at step 204, position data (slide position $z_0$) for the compression plate 26 is acquired from the holder section 28. Fig. 9 is a schematic diagram further schematically illustrating the relationship between the compression plate 26 and the imaging table 22 (the imaging face 20) illustrated in Fig. 8. At step 204, the slide position $z_0$ is acquired as the position data for the compression plate 26. Then, at step 206, the angle of inclination (angle $\theta$ in Fig. 9) of the compression plate 26 is acquired from the angle sensor 45.

At the next step 208, the separation t between the compression plate 26 and the imaging face 20 is computed according to Formula 1 and Formula 2 below for each of the positions where the AEC sensors 44 are provided, based on the compression plate 26 position data (slide position $z_0$), the angle of inclination $\theta$, and the y-axis direction position y of each AEC sensor 44. In the present exemplary embodiment, the y-axis direction position y of each of the AEC sensors 44 may be pre-stored in the storage section 47.

$$z_n = y_n \times \tan \theta \qquad (n = 1 \text{ to } 3) \qquad \text{Formula (1)}$$

$$t_n = z_0 + z_n = z_0 + y_n \times \tan \theta \qquad \text{Formula (2)}$$

At the next step 210, a corrected value of the detection value for each of the AEC sensors 44 is computed based on the computed separation t. For example, in the present exemplary embodiment, a correction coefficient is computed using Formula 3 below for each of the AEC sensors 44 by performing normalization of the computed separations t with an average value T.

$$\text{Correction coefficient} = t_n / T \qquad \text{Formula (3)}$$

The method for computing the correction coefficient is not limited to the above, and any other computation method may be employed. Alternatively, a configuration be made such that a correspondence relationship between the separations t and the correction coefficient is obtained in advance through testing, this correspondence relationship is stored in the storage section 47, and correction coefficients is determined according to the separations t based on this correspondence relationship.

At the next step 212, the computed correction coefficients are stored in the storage section 47.

At the next step 214, pre-irradiation is performed by the radiation irradiation section 24 prior to main imaging (prior to imaging of the desired radiographic image at step 108 in Fig. 6). The amount of transmitted radiation by pre-irradiation is detected by each of the AEC sensors 44 after the radiation irradiated from the radiation source 30 has passed through the compression plate 26 and the breast N. Detection values are output from each of the AEC sensors 44. At the next step 216, the detection values of each of the AEC sensors 44 are acquired, and at step 218 the acquired detection values of each of the AEC sensors 44 are corrected employing the correction coefficients obtained by the above processing so as to perform weighting according to the thickness of the breast N. Specifically, the weighting may be performed by multiplying each of the obtained detection values by the corresponding correction coefficients.

At step 220, imaging conditions for performing main imaging at an appropriate radiation amount are determined based on the corrected detection values (corrected values), and the imaging conditions are instructed to the radiation irradiation section 24. Then, the present processing ends. A correspondence relationship between the corrected values and imaging conditions (or the radiation amount) may be stored in advance in the storage section 47, and the imaging conditions may be determined with reference to this correspondence relationship. In the present exemplary embodiment, imaging conditions (such as the radiation amount) is varied or adjusted by adjusting at least one condition selected from the type of the target 30C of the radiation source 30, the type of the filter 24A, the tube voltage (the voltage between the cathode 30B and the target 30C), the tube current, and the radiation irradiation duration.

In the radiographic imaging device 10 of the present exemplary embodiment, main imaging is performed in the radiographic imaging processing (see step 108 in Fig. 6) under the imaging conditions set in the imaging condition setting processing, and an image expressing a radiographic image obtained from the radiation detector 42 is subjected to radiographic image correction processing using the correction coefficients described above, thereby improving the precision and image quality of the generated radiographic image. Fig. 10 is a flowchart illustrating an example of a flow of this radiographic image correction processing.

At step 300, image data expressing a radiographic image is acquired from the radiation detector 42. At the next step 302, the correction coefficients stored in the storage section 47 at step 212 of the imaging condition setting processing described above is read. At step 304, the image data is corrected using the read correction coefficients, and then the present processing is ended. The correction method for the image data is not particularly limited. For example, a correction method may be adopted in which regions (pixels 70) corresponding to each of the AEC sensors 44 are determined in the radiographic image, and the image data is corrected by using a corresponding correction coefficient for each of these regions. More specifically, the correction method may include multiplying the image data by the corresponding correction coefficient for each of these regions.

As explained above, in the radiographic imaging device 10 of the present exemplary embodiment, the angle sensor 45 detects the angle of inclination $\theta$ of the compression plate 26 after the breast N has been compressed by the compression plate 26. The separation $t_n$ at each of the positions where the AEC sensors 44 are provided is then computed based on the detected angle of inclination $\theta$, the slide position $z_0$ and the y-axis direction position $y_n$ (n = 1 to 3) of the compression plate 26. The correction coefficients are determined according to the computed separation $t_n$ and stored in the storage section 47. Pre-irradiation is performed by the radiation irradiation section 24, detection values of the radiation amount (transmitted amount) that has passed through the breast N are acquired from each of the AEC sensors 44, and each of the acquired detection values are respectively corrected by the correction coefficients to obtain corrected values. Instruction is made to the radiation source 30 to perform imaging under imaging conditions determined based on these corrected values. Imaging of the breast N to obtain a desired radiographic image is performed after adjustment of the radiation source 30.

As described above, in the present exemplary embodiment, even in a condition in which the thickness of the breast N is non-uniform due to the inclination of the compression plate 26, the thickness of the breast N is estimated from the separation $t_n$ between the compression plate 26 and the imaging face 20 at each of the positions where the AEC sensors 44 are provided, and the detection values of the AEC sensors 44 are corrected with the correction coefficients that take into account the non-uniform thickness of the breast N. Therefore, it is possible to improve the detection precision of the transmitted radiation amount by the AEC sensors 44. Further, in a case in which the transmitted radiation amount is relatively small, determination can be made easily as to whether the transmitted radiation amount is small due to the thickness of the breast N being thick, or due to the presence of tissue through which radiation is not readily transmitted.

The quality of radiographic images can also be improved due to correcting the obtained radiographic image using the correction coefficients.

Although the above exemplary embodiment estimates the thickness of the breast N using the angle of inclination $\theta$ of the compression plate 26 in a direction from the holder section 28 towards the chest wall 25, there are also cases in which the compression plate 26 is inclined in the left-right direction of the subject W. For example when the AEC sensors 44 are disposed as shown in Fig. 4, there are cases in which the compression plate 26 tilts such that the separations t at the AEC sensors $44_{11}$, $44_{12}$, $44_{13}$ are relatively large (the breast N is thicker) and the separations t become gradually smaller (the breast N is thinner) towards the AEC sensors $44_{31}$, $44_{32}$, $44_{33}$. Reversely, there are also cases in which the compression plate 26 tilts such that the separations t of the AEC sensors $44_{11}$, $44_{12}$, $44_{13}$ are relatively small (the breast N is thinner) and the separations t become gradually larger (the breast N is thicker) towards the AEC sensors $44_{31}$, $44_{31}$, $44_{33}$. There are also cases in which the compression plate 26 tilts such that the central portion of the compression plate 26 is at the highest position, and both of the edge portions of the compression plate 26 are at the lowest positions. In such cases, the separations t are relatively large (the breast N is thicker) at the AEC sensors $44_{21}$, $44_{22}$, $44_{23}$ and the separations t at the AEC sensors $44_{11}$, $44_{12}$, $44_{13}$ and the separations t at the AEC sensors $44_{31}$, $44_{32}$, $44_{33}$ are smaller (the breast N is thinner).

In such cases, a detection sensor (such as an angle sensor) may be provided to detect a tilt angle (angle of inclination $\varphi$) of the compression plate 26 in the left-right direction of the subject W, and the correction coefficients may be computed based on the angle of inclination $\theta$ of the above exemplary embodiment and the angle of inclination $\varphi$. Alternatively, a correspondence relationship between the angles of inclination $\theta$ and $\varphi$ and the correction coefficient may be stored, and correction coefficients may be determined based on this correspondence relationship.

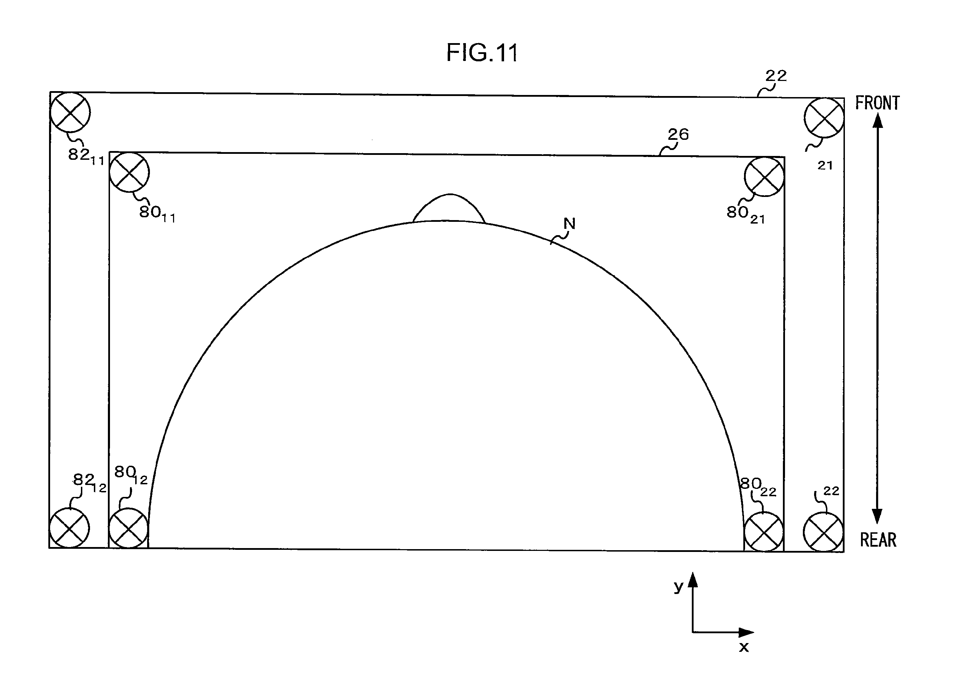

Further, although in the above exemplary embodiment, the angle of inclination $\theta$ of the compression plate 26 is detected, the separation $t_n$ is computed based on the angle of inclination $\theta$, and the separation $t_n$ is estimated as the thickness of the breast N, there is no limitation thereto. The separations t between the compression plate 26 and the imaging face 20 may be directly detected. For example, sensors (such as sensors $80_{11}$, $80_{12}$, $80_{21}$, $80_{22}$ in Fig. 11) may be provided at the four corners of the compression plate 26 to detect the separations t. In a case in which infrared sensors are provided, the separations t may be detected by irradiating infrared radiation from the infrared sensors and detecting the infrared radiation bouncing back from the imaging face 20 of the imaging table 22. Alternatively, light emitting devices and light receiving devices may be provided on the compression plate 26 and the imaging table 22 respectively, and the separations t may be detected based on light reception signals of the light receiving devices upon receiving light (signals) emitted from the light emitting devices. In such cases, as shown in Fig. 11, the sensors 80 (light emitting devices or light receiving devices) may be provided at the four corners of the compression plate 26, and the sensors $82_{11}$, $82_{12}$, $82_{21}$, $82_{22}$ (light receiving devices or light emitting devices) may be respectively provided at positions correspond to the positions of the four corners of the compression plate 26 such as the four corners of the imaging table 22 or positions below the compression plate 26. Note that the positions where the sensors 80, or the combination of the sensors 80 and the sensors 82, are provided are not limited to the four corners of the compression plate 26, and they may be provided at other positions, or one or more sets of the sensors 80, or the combination of the sensors 80 and the sensors 82, may be further provided. Although the detection precision of the separations t is improved by providing sensors at plural positions, there is a concern regarding affecting radiographic imaging. In such cases, the positions and number of the sensors 80, or the combination of the sensors 80 and the sensors 82, may be determined in considerations of the characteristics of the sensors 80 and the sensors 82, or the desired image quality of radiographic images. In cases in which the compression plate 26 is formed from a flexible substrate or formed in a sheet shape, which may readily deform and incline along plural directions by following the shape of the breast N during compression of the breast N, more appropriate correction coefficients may be obtained by directly detecting the separations t.

In the present exemplary embodiment, estimation is made that the separation t = the thickness of the breast N; however there are cases in which gaps are present between the breast N and the compression plate 26 and/or the breast N and the imaging table 22 at the leading end portion of the breast N (see the separation $t_1$ at the AEC sensors $44_{11}$, $44_{21}$, $44_{31}$ in Fig. 8). There are also cases in which the breast N is not present at the positions where the AEC sensors 44 are provided if the breast N is small. Such cases can be accommodated by configuring such that correction coefficients corresponding to cases in which gaps are present are obtained and stored in advance through testing, for example, and providing pressure sensors and/or temperature sensors or the like to the compression plate 26 and the imaging table 22 at positions where gaps may be present. In this way, determination may be made such that a gap is present if the pressure detected by the pressure sensor is small, or the temperature detected by the temperature sensor is low, and the correction coefficients corresponding to the cases in which a gap is present may be acquired. Further, determination may be made such that the breast N is not present and that a space is present if the pressure is further smaller or the temperature is further lower than in the cases in which a gap is present, and a corresponding correction coefficient may be determined therefor.

In the present exemplary embodiment, explanation has been given for a configuration in which nine AEC sensors 44 are provided; however, there is no limitation thereto. The detection precision of the transmitted radiation amount is further improved by determining the positions of the AEC sensors 44 by taking into consideration the cases in which the thickness of the breast N is not uniform.

Second Exemplary Embodiment

Explanation follows regarding a case in which the pixels 70 of the radiation detector 42 are employed as the detection sensors of radiation amount. Since the present exemplary embodiment includes configurations and operations that are substantially the same as those of the first exemplary embodiment, these configurations and operations are indicated as such and detailed explanation thereof is omitted.

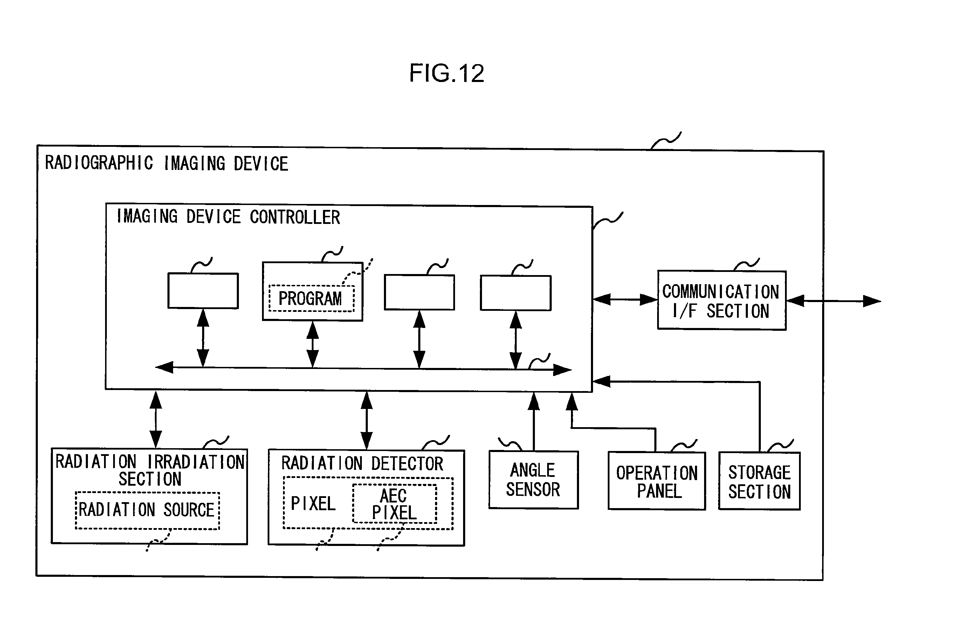

In the present exemplary embodiment, some of the pixels among the pixels 70 of the radiation detector 42 are employed as detection sensors (detection sensors 71 in Fig. 12) that detect a radiation amount. In the present exemplary embodiment, the pixels that serve as the detection sensors 71 can be set as desired among the pixels 70 of the radiation detector 42. Hereinafter, the pixels among the pixels 70 that serve as the detection sensors 71 are referred to as AEC pixels 70. In the present exemplary embodiment, the density of the AEC pixels for detecting a radiation amount is determined according to the thickness of the breast N, which is estimated from the angle of inclination $\theta$ of the compression plate 26. The density of the AEC pixels 70 is lowered at portions where the thickness of the breast N is thinner and data is much more likely to be obtained (the transmitted radiation amount may be high). In contrast, the density of the AEC pixels is heightened in order to increase the S/N ratio at portions where the thickness of the breast N is thicker and data is less likely to be obtained (the transmitted radiation amount may be low). The density of the radiation amount detection pixels $70_{AEC}$ is thus made to differ according to the thickness of the breast N. This enables both pre-exposure that takes into consideration the thickness of the breast N and faster detection of radiation amount to be achieved.

Detailed explanation follows regarding a specific example of the present exemplary embodiment.

Example 1

Fig. 12 is a function block diagram illustrating a configuration of a radiographic imaging device 10 of the present example. As shown in Fig. 12; the radiographic imaging device 10 of the present example has substantially the same configuration as the radiographic imaging device 10 of the first exemplary embodiment (illustrated in Fig. 5), except that the AEC sensors 44 of the radiographic imaging device 10 of the first exemplary embodiment are not provided. Instead, some of the pixels 70 (AEC pixels 70) of the radiation detector 42 are employed as detection sensors 71 for detecting radiation amount. In this example, one AEC pixel 70 corresponds to one detection sensor 71. That is, the number of the AEC pixels 70 and the number of the detection sensors 71 are equal.

The storage section 47 in the present example stores in advance separations (threshold values) $t_1$, $t_2$ instead of the data stored in the storage section 47 in the first exemplary embodiment. Since the radiographic imaging device 10 and the radiation detector 42 of the present example have substantially the same configurations as that of the radiographic imaging device 10 illustrated in Fig. 1 and the schematic configuration of the radiation detector 42 illustrated in Fig. 3 of the first exemplary embodiment, detailed explanation thereof is omitted.

An overall flow of the radiographic imaging processing by the radiographic imaging device 10 of the present example is substantially the same as the flow of the radiographic imaging processing of the first exemplary embodiment (see Fig. 6), except that the imaging condition setting processing accompanying the pre-irradiation at step 106 and the radiographic image correction processing performed during the radiographic imaging processing at step 108 in the present example differ from those of the first exemplary embodiment. Detailed description accordingly follows regarding these processing.

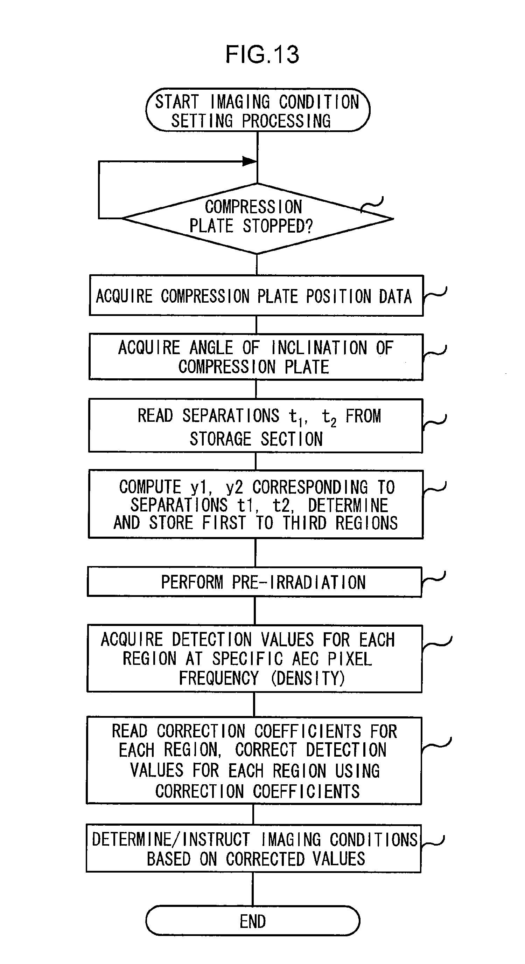

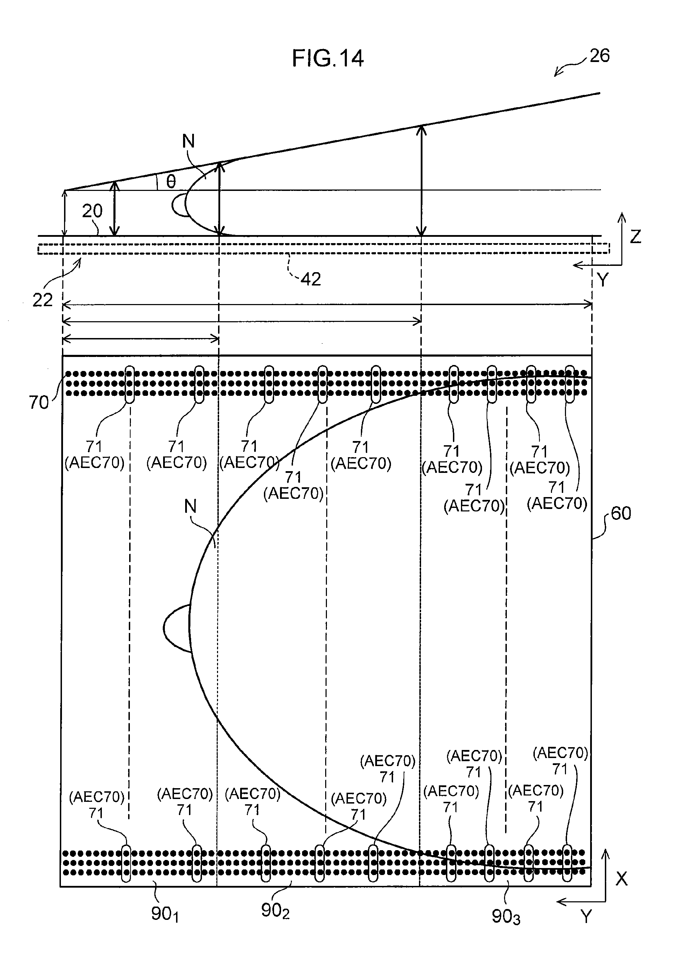

Firstly, explanation follows regarding the imaging condition setting processing of the present example. Fig. 13 is a flow chart of the imaging condition setting processing in the present example. Fig. 14 is a schematic diagram illustrating the arrangement of the detection sensors 71 (the AEC pixels 70) in the present example.

At step 402 to step 406, substantially the same processing is performed as in the imaging condition setting processing of the first exemplary embodiment (see step 202 to step 206 in Fig. 7). Determination is made as to whether or not movement of the compression plate 26 has stopped, and if it is determined that the movement has stopped, position data (the slide position $z_0$) of the compression plate 26 is acquired from the holder section 28. The angle of inclination $\theta$ of the compression plate 26 is also acquired from the angle sensor 45.

At the next step 408, the separation $t_1$ and the separation $t_2$ are read from the storage section 47.

At the next step 410, a first region to a third region are determined based on the position data (slide position $z_0$), the angle of inclination $\theta$ of the compression plate 26, and the separation $t_1$ and the separation $t_2$. Data indicating the first region to the third region are stored in the storage section 47.

In the present example, a pixel region, where the pixels 70 are provided on the face of the radiation detector 42 that is irradiated with radiation, is divided into three regions (a first region $90_1$ to a third region $90_3$) according to the thickness of the breast N (the inclination of the compression plate 26). In the present example, as shown in Fig. 14, the first region $90_1$ to the third region $90_3$ are determined according to a separation t (y) from the imaging face 20 to the compression plate 26 as follows.

$$
\begin{aligned}
&\text{First region } 90_1: &&\text{to } (z_0) \le t\,(y) < t_1 \\
&\text{Second region } 90_2: &&t_1 \le t\,(y) < t_2 \\
&\text{Third region } 90_3: &&t_2 \le t\,(y) \\
&y_n \text{ of } n^{\text{th}} \text{ region } 90_n &&= (t_y - z_0)\,/\,\tan\theta \quad (n = 1 \text{ to } 3)
\end{aligned}
$$

The third region $90_3$ is a region of the pixel region excluding the first region $90_1$ and the second region $90_2$, and $y_n$ is determined as above by acquiring $\tan\theta$ and the slide position $z_0$.

After the first region $90_1$ to the third region $90_3$ have been determined accordingly and stored in the storage section 47, pre-irradiation is performed at the next step 412 by the radiation irradiation section 24 prior to main imaging. In this pre-irradiation, radiation irradiated from the radiation source 30 reaches the radiation detector 42 after passing through the compression plate 26 and the breast N. Charges (or electrical signal) are accumulated in each of the pixels 70 of the radiation detector 42 according to the radiation amount.

At the next step 414-1, detection values (electrical signals corresponding to the accumulated charges) are acquired from the AEC pixels 70 provided at a specific frequency in each of the regions (the first region $90_1$ to the third region $90_3$).

In the present example, regions are determined based on the thickness of the breast N, and the frequency (density) of the AEC pixels 70 that serve as the detection sensors 71 is predetermined for each of the regions. The frequency (density) of the AEC pixels 70 is determined based on the thickness of the breast N. Specifically, a table indicating the correspondence between the thickness of the breast N and the density of the AEC pixels 70 may be pre-stored in, for example, the storage section 47 or the ROM 52. Then, the regions  corresponding to the actual thickness of the breast N are determined and the corresponding density is applied for each of the regions. For example, in the first region $90_1$, which corresponds to a region where the thickness of the breast N will be relatively thin, one pixel of every nine pixels in a row along the y direction is selected as an AEC pixel 70. In the second region $90_2$, which is a region where the thickness of the breast N will be an intermediate value, one pixel of every seven pixels in a row along the y direction is selected as an AEC pixel 70. In the third region $90_3$, which corresponds to a region where the thickness of the breast N will be relatively thick, one pixel of every five pixels in a row along the y direction is selected as an AEC pixel 70. Accordingly, in the configuration of the present example, the density in the y direction of the AEC pixels 70 increases as the thickness of the breast N increases. Further, single pixels from among the plural pixels 70 arrayed at positions with different breast N thicknesses are employed as the AEC pixels 70. That is, the AEC pixels 70 are selected from sets of the pixels 70 arrayed in the y direction, since the thickness of the breast N is substantially constant for the pixels 70 arrayed along the x direction. In this regard, the density of the AEC pixels 71 in the x direction is made consistent (e.g., every three pixels) because the thickness of the breast N is substantially constant. The pixels 70 at the y direction positions selected as above are employed as the AEC pixels 70. The selection method of a single pixel that serves as the AEC pixel 70 from a set of the pixels 70 arrayed along the y direction may be predetermined, but is not particularly limited. Further, embodiments are not limited to the configuration of the present example, in which all of the pixels 70 in the x direction are selected in sets of three pixels as the AEC pixels 70 for respective detection sections 71. For example, sets of three pixels in the x direction may be selected as the AEC pixels 70 with a uniform interval between respective sets of three pixels. The number of the AEC pixels 70 in the x direction or the interval therebetween may be stored in the storage section 47.

The method for acquiring the detection values from the AEC pixels 70 (detection sensors 71) of the radiation detector 42 is not particularly limited. In a case in which the signal lines 76 are provided along the y direction and the scan lines 78 are provided along the x direction, signals for switching ON the TFT switches 72 may be applied in sequence to only those scan lines 78 that correspond to the positions of the AEC pixels 70 in the y direction, thereby driving the TFT switches 72 and reading the accumulated charges (electrical signals) from the sensor portions 74 to the signal lines 76. The electrical signals read through the signal lines 76 are acquired as the detection values by the controller 66 through the signal detection circuit 64.

At the next step 416-1, predetermined correction coefficients are read from the  storage section 47 for each of the regions, and the detection values of each of the regions are corrected with the read correction coefficients. In the present example, the correction coefficients are predetermined for each of the regions according to the thickness of the breast N (the separation t). The correction coefficients according to the thickness of the breast N may be obtained in advance through testing and stored in the storage section 47. For example, the first region $90_1$ may have a correction coefficient based on the separation $t_0$, the second region $90_2$ may have a correction coefficient based on the separation $t_1$, and the third region $90_3$ have a correction coefficient based on the separation $t_2$. As another example, the first region $90_1$ may have a correction coefficient based on (separation to + separation $t_1$) / 2 that is an intermediate value between the separation to and the separation $t_1$, and the second region $90_2$ may have a correction coefficient based on (separation $t_1$ + separation $t_2$) / 2. In this case, the separation t at positions in the y direction in the vicinity of the base of the breast N is not known until the subject W is actually fixed by the compression plate 26, and cannot be obtained in advance. Accordingly, in the third region $90_3$, for example, a correction coefficient based on (the separation $t_2$ + a specific separation tw) may be employed.

In the present example, weighting is performed according to the thickness of the breast N by correcting the thus acquired detection values of the AEC pixels 70 (detection sensors 71) with the correction coefficients.

At the next step 418, imaging conditions for performing main imaging at an appropriate radiation amount are determined based on the corrected detection values (corrected values), the imaging conditions are instructed to the radiation irradiation section 24, and then the present processing is ended. In the present example, similarly as in the first exemplary embodiment, a correspondence relationship between corrected values and imaging conditions (or radiation amount) may be pre-stored in the storage section 47, and the imaging conditions may be determined with reference to this correspondence relationship. Variation and adjustment of the imaging conditions (such as radiation amount) in the present example may be performed similarly as in the first exemplary embodiment.

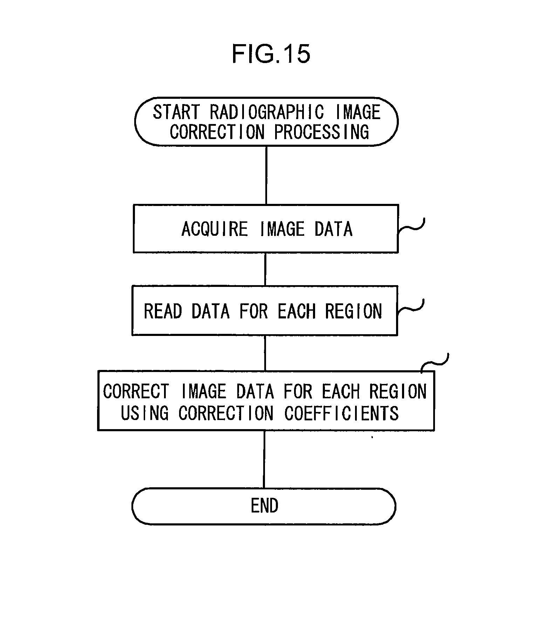

In the radiographic imaging device 10 of the present example, main imaging is performed under the imaging conditions set by the imaging condition setting processing (see Fig. 13) described above during radiographic imaging processing. Radiographic images obtained from the radiation detector 42 in this way are subjected to correction processing, in order to improve the precision and image quality of the generated radiographic images. Fig. 15 is a flow chart illustrating a flow of such correction processing.

At step 500, image data expressing a radiographic image is acquired from the radiation detector 42. At the next step 502, data that expresses each of the regions (the first region $90_1$ to the third region $90_3$) stored in the storage section 47 at step 410 of the imaging condition setting processing described above is read out. At the next step 504, the image data for each of the regions is corrected using the correction coefficients determined for each of the regions, and then the present processing is ended.

In the present example, a specific number of pixels 70 is determined for each of the regions that are provided according to the thickness of the breast N (the first region $90_1$ to the third region $90_3$), and a single pixel 70 of every specific number of pixels 70 arrayed along the y direction is employed as a detection sensor 71 (AEC pixel 70) for detecting radiation amount. Accordingly, the density of the AEC pixels 70 for detecting radiation amount can be varied according to the thickness of the breast N.

Example 2

A function block diagram of a configuration of the radiographic imaging device 10 of the present example is substantially the same as that of Example 1 (see Fig. 12) and, therefore, is omitted. Since the present example differs from Example 1 in the configuration of the AEC pixels 70 employed as the detection sensors 71 for detecting radiation amount, and in the manner of selecting thereof, explanation follows regarding these points. In the present example, each of the detection sensors 71 includes a set number of AEC pixels 70 that are arrayed in x direction and the set number is determined based on the thickness of the breast N.

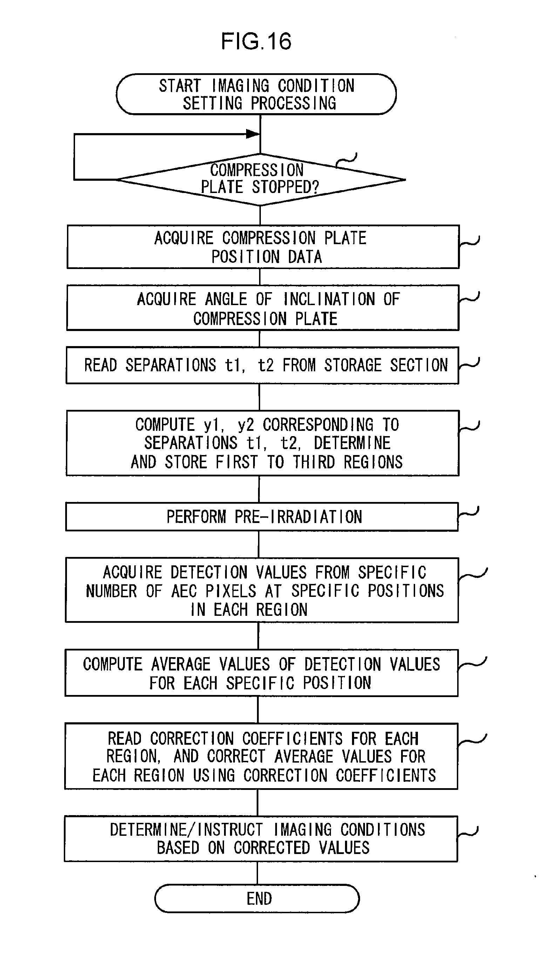

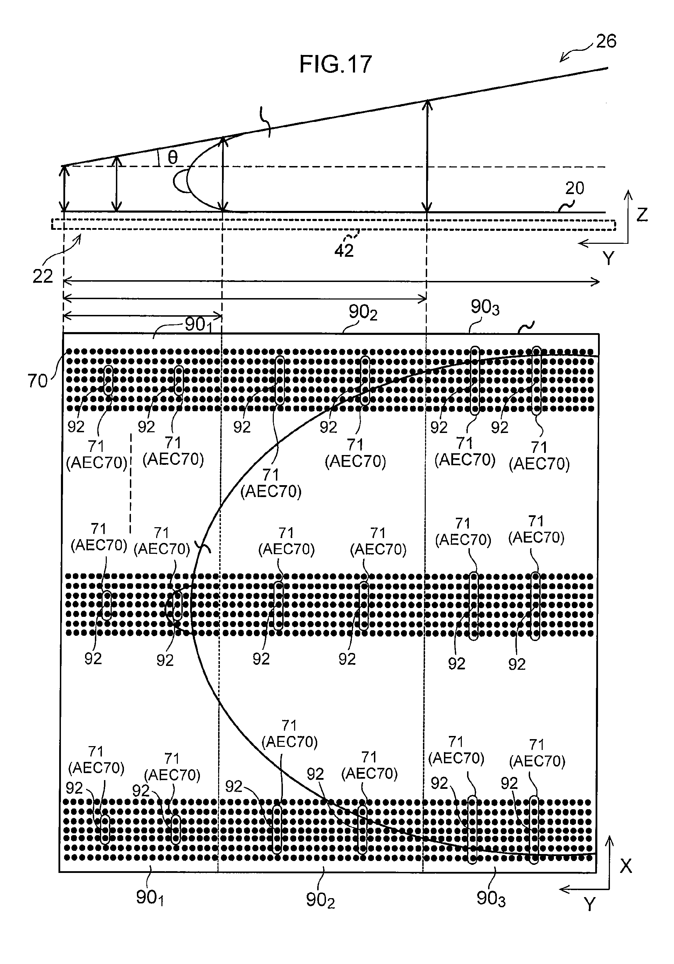

Fig. 16 is a flow chart illustrating an example of imaging condition setting processing of the present example. Since the imaging condition setting processing of the present example includes steps substantially the same as those of the imaging condition setting processing of Example 1 (see Fig. 13), these steps are allocated the same reference numerals, and detailed explanation thereof is omitted. Fig. 17 is a schematic diagram illustrating the arrangement of the detection sensors 71 (AEC pixels 70) of the present example.

Step 402 to step 412 of the imaging condition setting processing of the present example shown in Fig. 16 respectively correspond to step 402 to step 412 of the imaging condition setting processing of Example 1. Determination is made as to whether or not the movement of the compression plate 26 has stopped, and if it is determined that the movement has stopped, position data (the slide position $z_0$) of the compression plate 26 is acquired from the holder section 28. The angle of inclination $\theta$ of the compression plate 26 is also acquired from the angle sensor 45. The separation $t_1$ and the separation $t_2$ are read from the storage section 47, and a first region $90_1$ to a third region $90_3$ are determined based on the position data (the slide position $z_0$), and the angle of inclination $\theta$ of the compression plate 26, and the separation $t_1$ and the separation $t_2$. Data indicating the first region $90_1$ to the third region $90_3$ are stored in the storage section 47. Pre-irradiation is then performed by the radiation irradiation section 24 prior to main imaging. Accordingly, each of the pixels 70 of the radiation detector 42 accumulates charges (electrical signals) according to the radiation amount transmitted through the breast N during the pre-irradiation.

At the next step 414-2, detection values (the accumulated charges) are acquired from a number of AEC pixels 70 at specific positions (detection positions 92) for each of the regions (the first region $90_1$ to the third region $90_3$), where the number is determined based on the thickness of the breast N.

In the present example, the detection positions 92 for positioning the detection sensors 71 for detecting the radiation amount are predetermined. For example, as shown in Fig. 17, the detection positions 92 are predetermined in the imaging plane such that the intervals between the center positions of the detection positions 92 in the x direction and the intervals between the center positions of the detection positions 92 in the y direction are both uniform. The pixels 70 that correspond to the detection positions 92 are employed as the AEC pixels 70. Further, the number of the AEC pixels 70 in the x direction (the direction in which the thickness of the breast N is substantially the same), which are employed as the detection sensor 71 at the detection positions 92, is predetermined for each of the regions based on the thickness of the breast N.

In particular, a table indicating the correspondence of the locations of the detection positions 92, the thickness of the breast N and the number of AEC pixels 70 in the x direction may be pre-stored, for example, in the storage section 47 or the ROM 52. Then, the regions corresponding to the actual thickness of the breast N are determined and the corresponding number of AEC pixels 70 in the x direction is selected for each of the regions, such that a pixel positioned at the detection position 92 is the center position. Specifically, in the present example, three AEC pixels 70 in the x direction are selected in the first region $90_1$, which is a region where the thickness of the breast N is relatively thin, five AEC pixels 70 in the x direction are selected in the second region $90_2$ where the thickness of the breast N is an intermediate value, and seven AEC pixels 70 in the x direction are selected in the third region $90_3$.

There are no particular limitations to which of the pixels 70 corresponding to the detection positions 92 are employed as AEC pixels 70, but may be predetermined. For example, plural (the predetermined number of) pixels 70 that are arrayed along the x direction and centered on a single pixel 70 that is closest to the respective detection position 92 may be employed as the AEC pixels 70.

In this way, in the present example, the number of the AEC pixels 70 arrayed in the x direction increases as the thickness of the breast N increases. Further, an average value of the detection values of the plural AEC pixels that are used as one detection sensor 71 is employed as a detection value of the detection sensor 71. That is, the average value of the plural AEC pixels 70 provided at positions having the same thickness of the breast N is used as the detection value of the detection sensor 71.

In the present example, the pixels 70 arrayed in the x direction are employed as the AEC pixels 70 rather than the pixels 70 arrayed in the y direction, since the thickness of the breast N is different along the y direction.

At the next step 415-2, the average value of the detection values of the AEC pixels 70 are computed for each of the detection positions 92 (detection sensors 71). At the next step 416-2, predetermined correction coefficients for each of the regions are read from the storage section 47 and the average value of the detection values for each of the detection positions 92 are corrected using the correction coefficients for each of the regions. Thereby, the detection value of each of the detection sensors 71 is corrected. Similarly to Example 1, the correction coefficients are predetermined in advance according to the thickness of the breast N (the separation t) for each of the regions, and are stored in the storage section 47. In this way, weighting according to the thickness of the breast N is performed by correcting the acquired average values of the AEC pixels 70 for the detection positions 92 (detection sensors 71) with the correction coefficients.

The next step 418 corresponds to the step 418 of Example 1. That is, imaging conditions for performing main imaging at an appropriate radiation amount are determined based on the corrected average values (corrected values), the imaging conditions are instructed to the radiation irradiation section 24, and then the present processing is ended.

Thus, in the present example, the number of AEC pixels 70 is determined for each of the regions (the first region $90_1$ to the third region $90_3$) that are provided according to the thickness of the breast N, and the determined number of the AEC pixels 70 at each of the detection positions 92, where the number is based on the thickness of the breast N, are employed as one detection sensor 71 that detects a radiation amount. Further, an average value of the detection values of the AEC pixels 70 for each of the detection positions 92 (detection sensors 71) is used. In this way, it is possible to vary the density of the AEC pixels 70 that detect a radiation amount according to the thickness of the breast N.

Example 3

A function block diagram of a configuration of the radiographic imaging device 10 of the present example is substantially the same as that of Example 1 (see Fig. 12) and, therefore, is omitted. Since the present example differs from Example 1 in terms of the configuration of the AEC pixels 70 employed as the detection sensors 71 for detecting the radiation amount and of the manner of selecting the AEC pixels 70, explanation follows regarding these points. That is, each of the detection sensors 71 of the present example includes a set number of AEC pixels 70 that are arranged two-dimensionally.

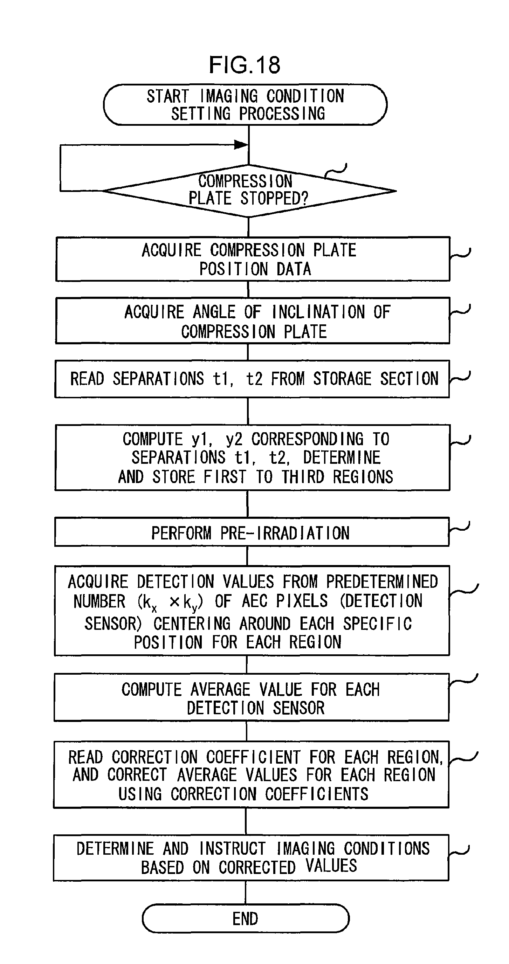

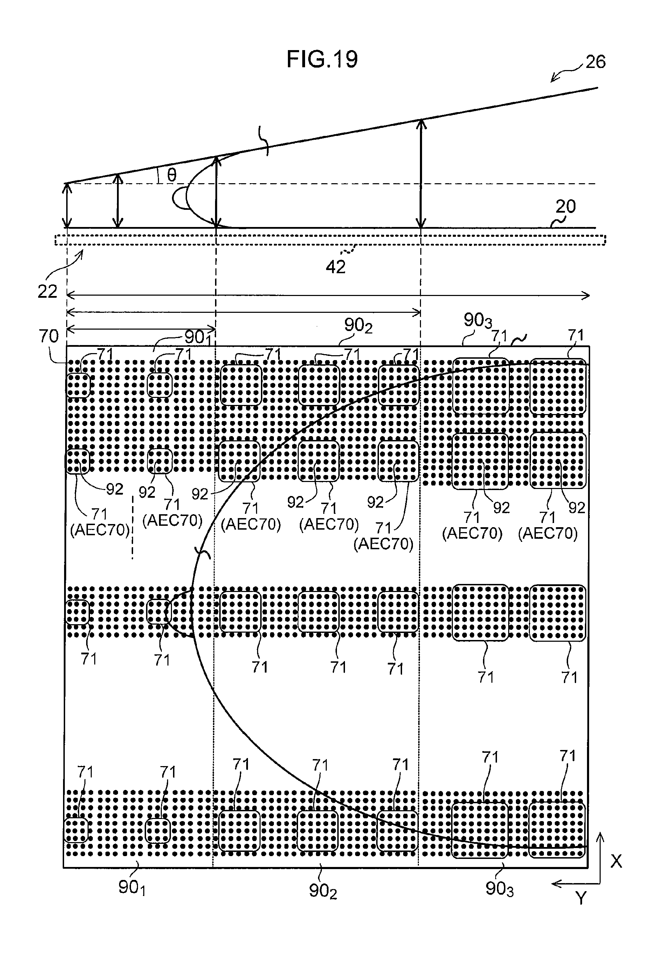

Fig. 18 is a flow chart illustrating an example of the imaging condition setting processing of the present example. Since the imaging condition setting processing of the present example includes steps that are substantially the same as those of the imaging condition setting processing of Example 1 (see Fig. 13), these steps are allocated the same reference numerals, and detailed explanation thereof is omitted. Fig. 19 is a schematic diagram illustrating the arrangement of the detection sensors 71 (AEC pixels 70) of the present example.

Step 402 to step 412 of the imaging condition setting processing of the present example shown in Fig. 18 respectively correspond to step 402 to step 412 of the imaging condition setting processing of Example 1. Determination is made as to whether or not the movement of the compression plate 26 has stopped, and if it is determined that the movement has stopped, position data (the slide position $z_0$) of the compression plate 26 is acquired from the holder section 28. The angle of inclination $\theta$ of the compression plate 26 is also acquired from the angle sensor 45. The separation $t_1$ and the separation $t_2$ are read from the storage section 47, and a first region $90_1$ to a third region $90_3$ are determined based on the position data (the slide position $z_0$), the angle of inclination $\theta$ of the compression plate 26, and the separation $t_1$ and the separation $t_2$. Data indicating the first region $90_1$ to the third region $90_3$ are stored in the storage section 47. Pre-irradiation is then performed by the radiation irradiation section 24 prior to main imaging. Accordingly, each of the pixels 70 of the radiation detector 42 accumulates charges (electrical signals) according to the radiation amount transmitted through the breast N during the pre-irradiation.

At the next step 414-3, detection values (the accumulated charges) are acquired from a predetermined number $(k_x \times k_y)$ of AEC pixels 70 respectively centering on each of the positions (detection positions 92) for each of the regions (the first region $90_1$ to the third region $90_3$).

In the present example, each of the detection sensors 71 for detecting the radiation amount includes the predetermined number $(k_x \times k_y)$ of AEC pixels 70 centering on the respective predetermined detection positions 92 or pixels 70. For example, as shown in Fig. 19, 10 pixels 70 (AEC pixels 70) in the x direction and the y direction having even intervals are predetermined as the pixels 70 (AEC pixels 70) that correspond to the detection positions 92. The manner of selecting the pixels 70 (AEC pixels 70) as the detection positions 92 is not limited to the above, and, for example, may be selected as in Example 2.

In particular, the locations of the detection positions 92, and the table indicating the correspondence of the thickness of the breast N and the numbers of AEC pixels 70 in the x and y directions may be pre-stored, for example, in the storage section 47 or the ROM 52. Then, the regions corresponding to the actual thickness of the breast N are determined and the corresponding numbers of AEC pixels 70 in the x and y directions are selected for each of the regions. Therefore, the number of AEC pixels 70 that are employed as one detection sensor 71 is different for each of the regions. For example, as shown in Fig. 19, the determined number of AEC pixels 70 is 9 ($k_x=k_y=3$, $3\times3=9$) in the first region $90_1$, which is a region where the thickness of the breast N will be relatively thin, 25 ($k_x=k_y=5$, $5\times5=25$) in the second region $90_2$ where the thickness of the breast N will be an intermediate value, and 49 ($k_x=k_y=7$, $7\times7=49$) in the third region $90_3$.

In the present example, the numbers ($k_x$ and $k_y$) of the AEC pixels 70 used as one detection sensor 71 for each of the regions are stored in advance in the storage section 47. Although $k_x$ and $k_y$ are set to be equal in the present example, configurations are not limited hereto and $k_x$ and $k_y$ may not be equal. Although the present Example shown in Fig. 19 has a configuration in which the detection sensors 71 are disposed so as not to overlap each other, embodiments are not limited hereto and, for example, some portions of the detection sensors 71 may overlap each other in cases in which the number of AEC pixels 70 that serve as one detection sensor 71 increases.

In this way, in the present example, the number of AEC pixels 70 that are arranged in a two-dimensional formation of ($k_x \times k_y$) increases as the thickness of the breast N increases. Further, similarly to Example 2, an average value of the detection values of the plural AEC pixels that are used as one detection sensor 71 is employed as a detection value of the detection sensor 71. That is, the average value of the plural AEC pixels 70 provided at each of the positions with the substantially same thickness of the breast N is employed as the detection value of the detection sensor 71.

At the next step 415-3, an average value of the detection values of the AEC pixels 70 is computed for each of the detection positions 92 (detection sensors 71). The average value of the detection values of the AEC pixels 70 that constitute one detection sensor 71 is treated as the detection value of the detection sensor 71.

At the next step 416-3, predetermined correction coefficients for each of the regions are read from the storage section 47 and the detection value of each of the detection positions 92 is corrected using the correction coefficients for each of the regions. Similar to the preceding examples, the correction coefficients are predetermined according to the thickness of the breast N (the separation t) for each of the regions, and are stored in the storage section 47. In this way, weighting according to the thickness of the breast N is performed by correcting the acquired average values of the AEC pixels 70 for the detection sensors 71 with the correction coefficients.

The next step 418 corresponds to step 418 of Example 1. That is, imaging conditions for performing main imaging at an appropriate radiation amount are determined based on the corrected average values (corrected values), the imaging conditions are communicated to the radiation irradiation section 24, and then the present processing is ended.

Thus, in the present example, each of the detection sensors 71 includes the AEC pixels 70 that are arranged in a two- dimensional formation. The detection positions 92 (center pixels) are predetermined, and the number of the AEC pixels 70 ($k_x \times k_y$) that constitute one detection sensor 71 is determined for each of the regions (the first region $90_1$ to the third region $90_3$) provided according to the thickness of the breast N. The determined number of the AEC pixels 70 for each of the detection sensors 71 increases as the thickness of the breast N increases. Further, an average value of the detection values of the AEC pixels 70 for each of the detection positions 92 (detection sensors 71) is used.

In the present example, the detection positions 92 (center pixels) are uniformly set across the imaging face 20. Therefore, the density of the detection positions 92 for each of the regions (i.e., the number of the detection positions 92 per unit area) is same across the plural regions (first region $90_1$ to third region $90_3$). Further, the density of the AEC pixels 70 within one detection sensor 71, when seen from the center pixel of the detection sensor 71, is the same across the plural regions. However, since the number of the AEC pixels 70 for one detection sensor 71 increases as the thickness of the breast N increases, the total number of the AEC pixels 70 is greater in a region having a greater thickness of the breast N. In this way, it is possible to vary the density of the AEC pixels 70 that detect a radiation amount according to the thickness of the breast N, such that the greater the thickness of the breast N, the greater the density of the AEC pixels 70.

Further, as the thickness of the breast N increases, the number of the adjacent AEC pixels, of which the detection values are used to obtain the average value, increases, and the detection precision of the transmitted radiation amount is improved.

Example 4

A function block diagram of an example of a configuration of the radiographic imaging device 10 of the present example is substantially the same as that of Example 1 (see Fig. 12) and, therefore, explanation thereof is omitted. In the present example, the AEC pixels 70 used for the detection sensors 71 that detect the radiation amount are determined based on a radiographic image obtained by pre-irradiation. Explanation is given below of a case in which a method for determining the AEC pixels 70 based on a radiographic image of the present example is applied to Example 1.

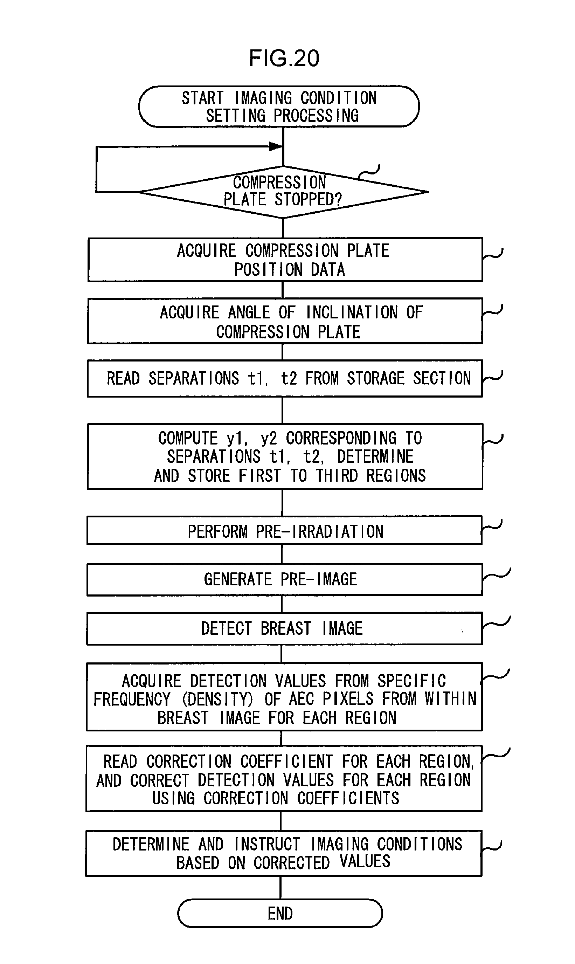

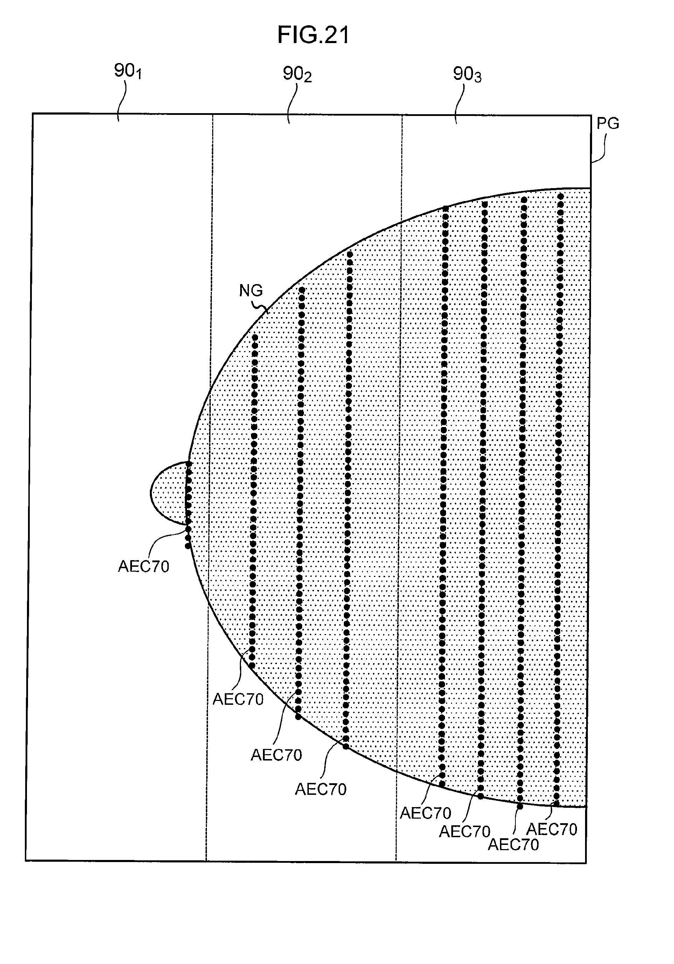

Fig. 20 is a flow chart illustrating an example of imaging condition setting processing of the present example. Since the imaging condition setting processing of the present Example includes steps that are substantially the same as those in the imaging condition setting processing of Example 1 (see Fig. 13), steps that are substantially the same are allocated the same reference numerals, and detailed explanation thereof is omitted. Fig. 21 is a schematic diagram illustrating the arrangement of the AEC pixels 70 that act as the detection sensors 71 in the present example. Fig. 19 schematically illustrates positions of the AEC pixels 70 (detection sensors 71) for each region (a first region $90_1$ to a third region $90_3$) on a pre-image PG (detailed explanation to follow) obtained by performing pre-irradiation.

As shown in Fig. 20, step 402 to step 412 of the imaging condition setting processing in the present example respectively correspond to step 402 to step 412 of the imaging condition setting processing of Example 1. Determination is made as to whether or not movement of the compression plate 26 has stopped, and if it is determined that the movement has stopped, position data (the slide position $z_0$) of the compression plate 26 is acquired from the holder section 28. The angle of inclination $\theta$ of the compression plate 26 is also acquired from the angle sensor 45. The separation $t_1$ and the separation $t_2$ are read from the storage section 47, and the first region $90_1$ to the third region $90_3$ are determined based on the position data (the slide position $z_0$), the angle of inclination $\theta$ of the compression plate 26, and the separation $t_1$ and the separation $t_2$. Data indicating the first region $90_1$ to the third region $90_3$ are stored in the storage section 47. Pre-irradiation is then performed by the radiation irradiation section 24 prior to main imaging. Accordingly, each of the pixels 70 of the radiation detector 42 accumulates charges (an electrical signal) according to the radiation amount transmitted through the breast N during the pre-irradiation.

At the next step 413-41, the charges accumulated in each of the pixels 70 by the pre-irradiation are read, and a radiographic image is generated based on the pre-irradiation (referred to below as the pre-image PG, shown in Fig. 19).

At the next step 413-42, a breast image NG of the imaged breast N is detected in the generated pre-image PG. The detection method of the breast image NG is not particularly limited, and any generally performed image analysis method may be applied. In the present example, since the detected breast image NG in the pre-image PG is not used for purposes of medical diagnosis, but rather used for determining the AEC pixels 70, the precision of the image analysis may be relatively lower than that for medical diagnosis, provided that it is within a permissible range for determining the AEC pixels 70.

At the next step 415-4, detection values (electrical signals corresponding to the accumulated charges) are acquired from the ACE pixels 70 provided at a specific frequency inside the breast image NG for each of the regions (the first region $90_1$ to the third region $90_3$). In the present example, the detection values are acquired from the AEC pixels 70 that are selected at a frequency similar to that of example 1 for each of the regions. Here, as shown in Fig. 21, only those pixels 70 located at positions corresponding to the breast image NG are employed as the AEC pixels 70, and the detection values are acquired from these AEC pixels 70.

At the next step 416-4, predetermined correction coefficients for each of the regions are read from the storage section 47, and the detection values for each of the regions are corrected using the read correction coefficients.

The next step 418 corresponds to step 418 of Example 1. That is, imaging conditions for performing main imaging at an appropriate radiation amount are determined based on the corrected detection values (corrected values), the imaging conditions are instructed to the radiation irradiation section 24, and the present processing is then ended.

In this way, in the present example, the breast image NG is detected from the pre-image PG obtained by pre-imaging, and the AEC pixels 70 used for the detection sensors 71 are determined among the pixels 70 that are located at positions corresponding to the breast image NG. Thus, in the present example the detection precision of the transmitted radiation amount is improved due to employing the AEC pixels 70 located only at positions corresponding to the breast N.

Although explanation has been given above regarding a case in which the present example is applied to Example 1, there is no limitation thereto, and obviously the present example may also be applied to any other examples. For example, cases in which the present example is applied to Example 2 or Example 3 obviously fall within the scope of the invention.

In Example 1 to Example 4, explanation has been given regarding cases in which the compression plate 26 is tilted (by the angle of inclination $\theta$) along the front-rear direction of the subject W. However, similar configuration may also be made in cases in which the compression plate 26 is inclined (by the angle of inclination $\varphi$) in the left-right direction of the subject W. For example, as in the first exemplary embodiment, a detection sensor (such as an angle sensor) may be provided for detecting the angle of inclination $\varphi$), the pixel region may be divided into plural regions based on the angle of inclination $\varphi$, and the AEC pixels 70 may be determined among the pixels 70 so that the pixel density for each of the divided regions corresponds to the thickness of the breast N.

Embodiments are not limited to the configuration in which the AEC pixels 70 (detection sensors 71) are disposed discretely, as described in Example 1 to Example 3. Alternatively, the AEC pixels 70 may be disposed contiguously.

Embodiments are not limited to the configuration in which the pixel density is uniform along the x direction as described in Example 1 to Example 4. For example, the pixel density along the x direction may correspond to the pixel density along the y direction. For further example, the pixel density along the x direction may match the pixel density along the y direction.

In Example 1 to Example 4, a radiographic image is divided in three regions (the first region $90_1$ to the third region $90_3$) using two threshold values (the separations $t_1$ and $t_2$). However, the threshold values or the number of the divided regions are not limited thereto.

As described above, in the present exemplary embodiment, the density of the AEC pixels 70 for detecting a radiation amount is determined according to the thickness of the breast N (which is estimated from the angle of inclination $\theta$ of the compression plate 26) even in cases in which the thickness of the breast N is not uniform due to tilting of the compression plate 26. The detection precision of the transmitted radiation amount can be improved by varying the density of the AEC pixels 70 for detecting a radiation amount according to the thickness of the breast N, and it is possible to perform pre-exposure and main imaging of a radiographic image by taking into consideration the thickness of the breast N. Faster radiation amount detection can also be achieved since there is no need to employ all of the pixels 70 as the AEC pixels 71 (detection sensors 71).

Third Exemplary Embodiment

Explanation follows regarding a case in which the pixels 70 of the radiation detector 42 are employed as the detection sensors of the radiation amount. In contrast to the second exemplary embodiment in which part of the pixels 70 of the radiation detector 42 are employed as the AEC pixels 70, the present exemplary embodiment employs all of the pixels 70 as the AEC pixels 70. Since the present exemplary embodiment includes configurations and operations that are substantially the same as those of the preceding exemplary embodiments, these configurations and operations are indicated as such and detailed explanation thereof is omitted.

In the present exemplary embodiment, all of the pixels 70 of the radiation detector 42 are employed as the AEC pixels 70 that are used as the detection sensors 71. The number of AEC pixels for one detection sensor 71 for detecting a radiation amount is determined according to the thickness of the breast N, which is estimated from the angle of inclination $\theta$ of the compression plate 26. The number of AEC pixels is determined such that the greater the thickness of the breast N, the greater the number of AEC pixels for one detection sensor 71.

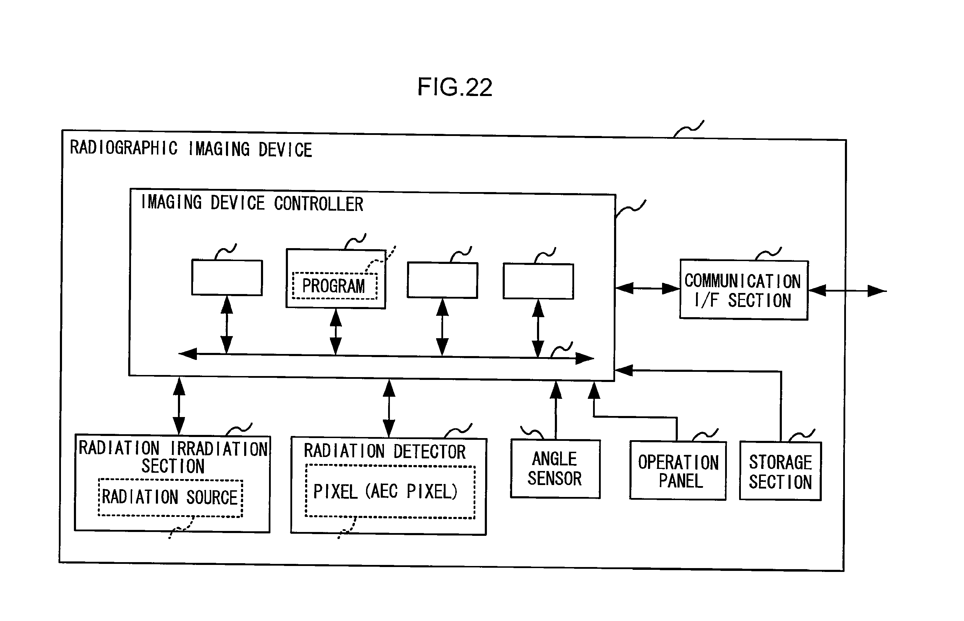

Fig. 22 shows a function block diagram of an exemplary configuration of the radiographic imaging device 10 of the present exemplary embodiment. As shown in Fig. 22, the radiographic imaging device 10 of the present exemplary embodiment has substantially the same configuration as the radiographic imaging device 10 of the first exemplary embodiment (Fig. 5), except that the AEC sensor 44 provided in the first exemplary embodiment is omitted. Further, all of the pixels 70 of the radiation detector 42 are employed as the AEC pixels 70 that are used as the detection sensors 71. In the present exemplary embodiment, a determined number ($k_x \times k_y$) of AEC pixels 70, where the number is determined based on the thickness of the breast N, serve as one detection sensor 71.

Similarly to the second exemplary embodiment, the storage section 47 stores in advance separations (threshold values) $t_1$ and $t_2$. Since the radiographic imaging device 10 and the radiation detector 42 of the present exemplary embodiment have substantially the same configurations as the radiographic imaging device 10 illustrated in Fig. 1 and the schematic configuration of the radiation detector 42 illustrated in Fig. 3 of the first exemplary embodiment, detailed explanation thereof is omitted.

An overall flow of the radiographic imaging processing by the radiographic imaging device 10 of the present exemplary embodiment is substantially the same as the flow of the radiographic imaging processing of the first exemplary embodiment (Fig. 6), except that the imaging condition setting processing accompanying the pre-irradiation at step 106 and the radiographic image correction processing performed during the radiographic imaging processing at step 108 in the present exemplary embodiment differ from those of the first exemplary embodiment. The radiographic image correction processing is similar to that in the second exemplary embodiment and, therefore, detailed description thereof is omitted. The imaging condition setting processing will be described in detail below.

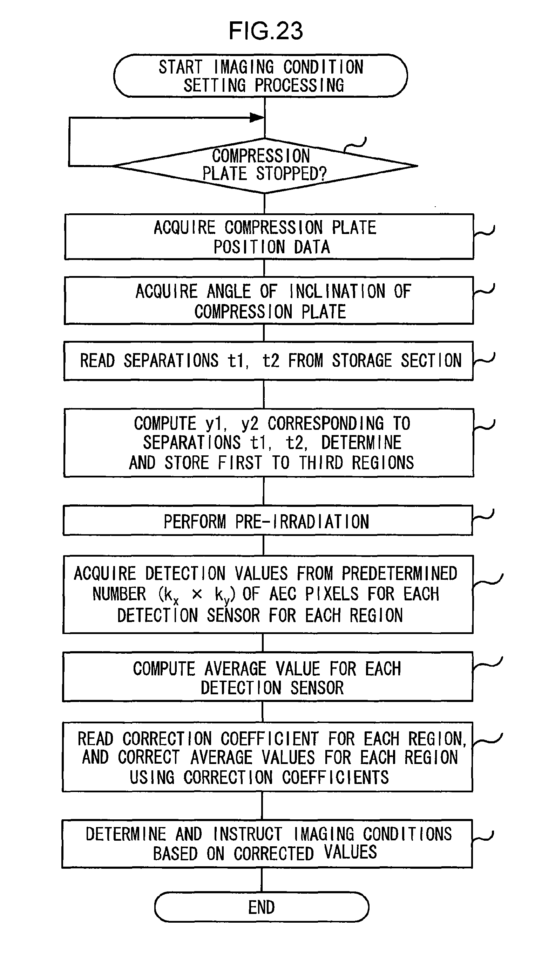

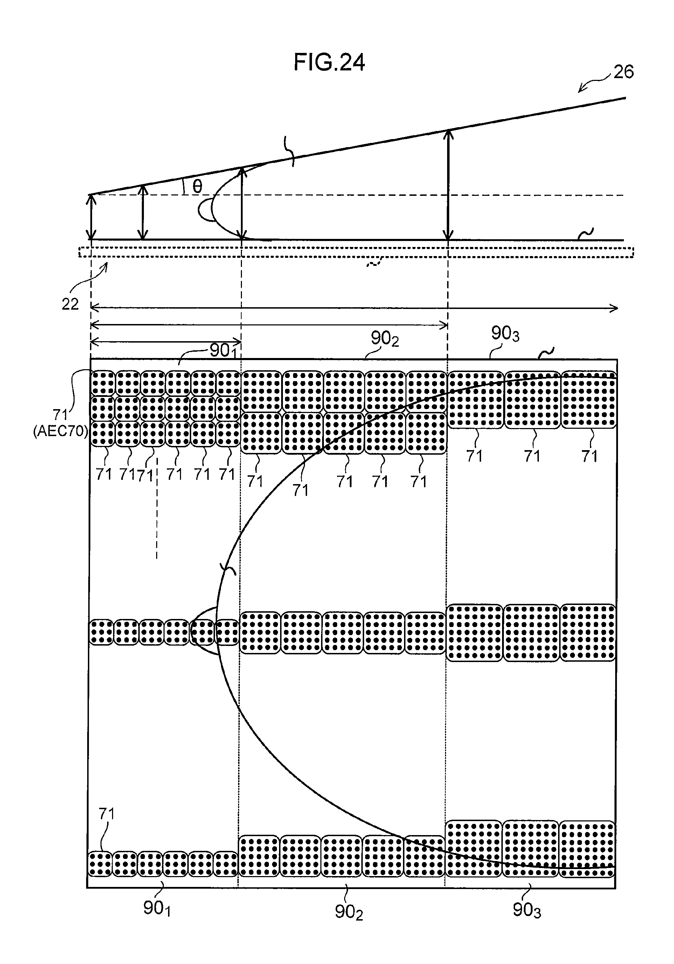

Fig. 23 is a flow chart illustrating an example of imaging condition setting processing of the present exemplary embodiment. Fig. 24 is a schematic diagram illustrating the arrangement of the AEC pixels 70 that act as the detection sensors 71 in the present exemplary embodiment.

At step 602 to step 606, substantially the same processing is performed as in the imaging condition setting processing of the first exemplary embodiment (see step 202 to step 206 in Fig. 7). Determination is made as to whether or not movement of the compression plate 26 has stopped, and if it is determined that the movement has stopped, position data (the slide position $z_0$) of the compression plate 26 is acquired from the holder section 28. The angle of inclination $\theta$ of the compression plate 26 is also acquired from the angle sensor 45.

At the next step 608, the separation $t_1$ and the separation $t_2$ are read from the storage section 47.

At the next step 610, a first region to a third region are determined based on the position data (slide position $z_0$), the angle of inclination $\theta$ of the compression plate 26, and the separation $t_1$ and the separation $t_2$. Data indicating the first region to the third region are stored in the storage section 47. The determination method for the first to third regions may be similar to that in the second exemplary embodiment.

At next step 612, pre-irradiation is performed by the radiation irradiation section 24 prior to main imaging. In this pre-irradiation, charges (or electrical signal) are accumulated in each of the pixels 70 of the radiation detector 42 according to the amount of radiation that has reached the radiation detector 42 after passing through the compression plate 26 and the breast N.

At the next step 614, detection values (electrical signals corresponding to the accumulated charges) are acquired from the AEC pixels 70 for each of the detection sensors 71 in each of the regions (the first region $90_1$ to the third region $90_3$).

In the present exemplary embodiment, the number ($k_x \times k_y$) of AEC pixels 70 that serve as one detection sensor 71 is determined for each of the regions. In particular, a table indicating the correspondence between the thickness of the breast N and the number ($k_x \times k_y$) of AEC pixels 70 may be pre-stored in the storage section 47 or the ROM 52, for example. Then, the regions corresponding to the actual thickness of the breast N are determined and the corresponding number of AEC pixels 70 is selected for each of the regions. Therefore, the number of AEC pixels 70 that serve as one detection sensor 71 differs in each of the regions. For example, as shown in Fig. 24, the predetermined numbers of the AEC pixels 70 are 9 ($k_x=k_y=3$, $3\times3=9$) in the first region $90_1$, which is a region where the thickness of the breast N will be relatively thin, 25 ($k_x=k_y=5$, $5\times5=25$) in the second region $90_2$ where the thickness of the breast N will be an intermediate value, and 49 ($k_x=k_y=7$, $7\times7=49$) in the third region $90_3$.

In the present exemplary embodiment, the predetermined numbers ($k_x$ and $k_y$) of AEC pixels 70 used as one detection sensor 71 in the respective regions are stored in advance in the storage section 47. Although $k_x$ and $k_y$ are set to be equal in the present exemplary embodiment, embodiments are not limited to this and $k_x$ and $k_y$ may not be equal.

At the next step 615, an average value of the detection values of the AEC pixels 70 is computed for each of the detection sensors 71. The average value of the detection values of the AEC pixels 70 that constitute one detection sensor 71 is treated as the detection value of the detection sensor 71.

At the next step 616, predetermined correction coefficients for each of the regions are read from the storage section 47 and the detection values of the detection sensors 71 are corrected using the correction coefficients for each of the regions. Similarly to the examples in the second exemplary embodiment, the correction coefficients are predetermined according to the thickness of the breast N (the separation t) for each of the regions, and are stored in advance in the storage section 47. In this way, weighting according to the thickness of the breast N is performed by correcting the acquired average values of the AEC pixels 70 for the detection sensors 71 with the correction coefficients.

In the next step 618, imaging conditions for performing main imaging at an appropriate radiation amount are determined based on the corrected detection values (corrected values), the imaging conditions are communicated to the radiation irradiation section 24, and then the present processing is ended. Similarly to the first exemplary embodiment, a correspondence relationship between the corrected values and the imaging conditions (or radiation amount) may be stored in the storage section 47, and the imaging conditions may be determined by referring to this correspondence relationship. Change or adjustment of the imaging conditions (such as the radiation amount) may be performed similarly to the first exemplary embodiment.

As described above, in the present exemplary embodiment, all of the AEC pixels 70 are used as the detection sensors 71. The number ($k_x \times k_y$) of the AEC pixels 70 for one detection sensor 71 is determined for each of the regions (the first region $90_1$ to the third region $90_3$) divided according to the thickness of the breast N, and the greater the thickness of the breast N, the greater the number of the AEC pixels 70 used as one detection sensor 71. Further, an average value of detection values of the AEC pixels 70 is used as the detection value for each of the detection positions 92 (detection sensors 71).

In the present example, the density of the AEC pixels 70 within one detection sensor 71, when seen from the center pixel of the detection sensor 71, is same across the plural regions (the first region $90_1$ to the third region $90_3$). However, since the number of the AEC pixels 70 per one detection sensor 71 increases as the thickness of the breast N is increases, the number of detection sensors 71 per unit area becomes smaller in regions of greater thickness of the breast N.

In the present exemplary embodiment, as the thickness of the breast N increases, the number of adjacent AEC pixels 70, of which the detection values are used to obtain the average value, increases, and the detection precision of the transmitted radiation amount is improved.

Embodiments are not limited to the configuration of the present exemplary embodiment in which all of the pixels 70 of the radiation detector 42 are used as the AEC pixels 70. For example, pixels 70 at the vicinity of the edges of the imaging face 20 may not be used as the AEC pixels 70, given that the detections sensors 71 are arranged without a space (for example, without a space in each of the regions), and this embodiment obviously falls within the scope of the invention. Further, similarly to Example 4 of the second exemplary embodiment, all of the pixels 70 that are located at positions corresponding to the breast image NG may be used as the AEC pixels 70, and the other pixels 70 that are not located at the positions corresponding to the breast image NG may not be used as the AEC pixels 70.

The detection sensors are not limited to the configuration described hitherto, such as the AEC sensors 44 that is separately provided in the first exemplary embodiment, the AEC pixels 70 selected from the pixels 70 in the second exemplary embodiment, and all of the pixels 70 in the third exemplary embodiment. For example, a detection sensor may be configured by incorporating radiation amount detection function into a portion of the radiation detector 42, and the present invention is also applicable to this configuration.

Embodiments are not limited to the embodiments described above in which the imaging site is the breast N, and the breast N is compressed by the compression plate 26 compresses the breast N. The imaging site is not limited to the breast N, provided that imaging of a radiographic image is performed in a state in which the imaging site is compressed.

The radiation employed in the radiographic imaging is not particularly limited, and, for example, X-rays or gamma rays may be applied.

The configuration of elements such as the radiographic imaging device 10, the radiation source 30 and the radiation detector 42 described in the above exemplary embodiments are examples thereof, and obviously modifications may be made according to circumstances within a range not departing from the spirit of the present invention. The flows of the radiographic imaging processing and imaging condition setting processing described in the exemplary embodiments are also examples thereof, and obviously modifications may be made according to circumstances, within a range not departing from the spirit of the present invention.

**Claims**

1.  A radiographic imaging device, comprising:

    a compression plate that compresses an imaging site of a subject between the compression plate and an imaging face of an imaging table, and that inclines with respect to the imaging face in accordance with the imaging site during the compression;
    a plurality of irradiation detection sections, each of which is provided at a different position on the imaging face and detects an irradiation amount of irradiated radiation; and
    a correction section that corrects detection results from the plurality of irradiation detection sections using correction coefficients that are based on the thickness of the imaging site compressed by the compression plate and on the positions of the plurality of irradiation detection sections.

2.  The radiographic imaging device of claim 1, further comprising:

    an estimation section that estimates the thickness of the imaging site compressed by the compression plate for each of the positions of the plurality of irradiation detection sections,
    wherein the correction section determines a correction coefficient for correcting each of the detection results from the plurality of irradiation detection sections based on the thickness of the imaging site estimated by the estimation section, and corrects each of the detection results from the plurality of irradiation detection sections using the determined correction coefficient.

3.  The radiographic imaging device of claim 2, wherein the estimation section detects a distance between the compression plate and the imaging face, and estimates the thickness of the imaging site according to the detected distance.

4.  The radiographic imaging device of claim 2 or claim 3, further comprising:

    an inclination detection section that detects the inclination of the compression plate with respect to the imaging face,
    wherein the estimation section further estimates the thickness of the imaging site based on the inclination of the compression plate detected by the inclination detection section.

5.  The radiographic imaging device of any one of claim 1 to claim 4, wherein the plurality of irradiation detection sections is provided along an inclination direction of the compression plate.

6.  The radiographic imaging device of claim 1, further comprising:

    a radiation detector comprising a plurality of pixels that accumulate charges in response to radiation and are disposed in a two-dimensional formation in a pixel region facing the imaging face;
    a region determination section that determines a plurality of regions in the pixel region based on a distance between the compression plate and the imaging face and on the inclination of the compression plate; and

an irradiation detection pixel determination section that determines, from among the plurality of pixels, irradiation detection pixels that are to be used as the irradiation detection sections, such that the irradiation detection pixels are disposed at a specific pixel density in each of the plurality of regions,

wherein the correction coefficient is predetermined for each of the plurality of regions, and the correction section corrects the detection results of the irradiation detection pixels for each of the regions using the predetermined correction coefficient.

7. The radiographic imaging device of claim 6, wherein the specific pixel density increases as the distance between the compression plate and the imaging face increases.

8. The radiographic imaging device of claim 6 or claim 7, wherein the irradiation detection pixel determination section determines, as the irradiation detection pixels for each of the plurality of regions, one pixel from each of a predetermined number of pixels arrayed along an inclination direction of the compression plate, the predetermined number being determined for each of the regions.

9. The radiographic imaging device of claim 6 or claim 7, wherein:

the irradiation detection pixel determination section determines, as the irradiation detection pixels for each of specific positions in the plurality of regions, a predetermined number of pixels, the predetermined number being determined for each of the regions; and
the correction section corrects an average value of detection results of the irradiation detection pixels for each of the specific positions using the correction coefficient.

10. The radiographic imaging device of claim 9, wherein the irradiation detection pixel determination section determines, as the irradiation detection pixels for each of the specific positions, the predetermined number of pixels, which are arrayed along a direction different from the inclination direction.

11. The radiographic imaging device of claim 9, wherein the irradiation detection pixel determination section determines, as the irradiation detection pixels, the predetermined number of pixels, which are arranged in a two-dimensional formation centering on the specific positions.

12. The radiographic imaging device of any one of claim 6 to claim 11, wherein:

the irradiation detection pixel determination section identifies a position of the imaging site on the imaging face based on a radiographic image in which the imaging site is imaged by the radiation detector, and determines the irradiation detection pixels from the plurality of pixels provided at positions corresponding to the identified position of the imaging site.

13. The radiographic imaging device of claim 1, further comprising:

a radiation detector comprising a plurality of pixels disposed in a two-dimensional formation in a pixel region facing the imaging face, the plurality of pixels being used for imaging of a radiographic image by accumulating charges in response to radiation and being used as irradiation detection pixels;
a region determination section that determines a plurality of regions in the pixel region based on a distance between the compression plate and the imaging face and on the inclination of the compression plate; and
a detection section determination section that determines, for each of the plurality of regions, a plurality of detection sections including a predetermined number of pixels, the predetermined number being determined for each of the regions and increasing as the thickness of the imaging site increases,
wherein the correction coefficient is predetermined for each of the plurality of regions, and the correction section corrects average values of the detection results of the detection sections for each of the regions using the predetermined correction coefficient.

14. The radiographic imaging device of any one of claim 1 to claim 13, further comprising an adjustment section that adjusts at least one of a radiation amount or radiation characteristics of radiation for imaging a radiographic image of the imaging site, based on the detection results from the plurality of irradiation detection sections that have been corrected by the correction section.

15. The radiographic imaging device of claim 14, wherein an object of adjustment by the adjustment section is at least one object selected from the group consisting of a tube voltage of a radiation source that generates the radiation, a tube current of the radiation source, an irradiation duration of the radiation source, a type of target for generating bremsstrahlung radiation that is provided at the irradiation source, and a type of filter provided between the radiation source and the subject.

16. The radiographic imaging device of any one of claim 1 to claim 15, further comprising an image correction section that corrects an imaged radiographic image using the correction coefficient determined by the correction section.

17. The radiographic imaging device of any one of claim 1 to claim 16, further comprising a storage section that stores the correction coefficient.

18. A radiographic imaging method in a radiographic imaging device comprising:

compressing an imaging site of a subject between a compression plate and an imaging face of an imaging table while inclining the compression plate with respect to the imaging face in accordance with the imaging site;
detecting an irradiation amount of irradiated radiation using a plurality of irradiation detection sections, each of which is provided at a different position on the imaging face; and
correcting detection results from the plurality of irradiation detection sections using correction coefficients that are based on the thickness of the imaging site compressed by the compression plate and on the positions of the plurality of irradiation detection sections.

19. The radiographic imaging method of claim 18, further comprising adjusting at least one of a radiation amount or radiation characteristics of radiation with which the examination subject is irradiated, based on the corrected detection results of the plurality of irradiation detection sections.

20. A computer readable storage medium stored with a program that causes a computer to execute radiographic imaging processing, the radiographic imaging processing comprising:

compressing an imaging site of a subject between a compression plate and an imaging face of an imaging table while inclining the compression plate with respect to the  imaging face in accordance with the imaging site;
detecting an irradiation amount of irradiated radiation using a plurality of irradiation detection sections, each of which is provided at a different position on the imaging face; and
correcting detection results from the plurality of irradiation detection sections using correction coefficients that are based on the thickness of the imaging site compressed by the compression plate and on the positions of the plurality of irradiation detection sections.

# FIG.1

REAR　　　　　　　　　　FRONT

# FIG.2

MOLYBDENUM

RHODIUM

ALUMINUM

SILVER

EP 2 649 942 A1

EP 2 649 942 A1

**FIG.3**

FIG.4

# FIG.5

RADIOGRAPHIC IMAGING DEVICE 10

IMAGING DEVICE CONTROLLER 48

| CPU 50 | ROM 52 / PROGRAM 53 | RAM 54 | HDD 56 |

COMMUNICATION I/F SECTION 49

57

RADIATION IRRADIATION SECTION 24
RADIATION SOURCE 30

RADIATION DETECTOR 42

AEC SENSOR 44

ANGLE SENSOR 45

OPERATION PANEL 46

STORAGE SECTION 47

EP 2 649 942 A1

## FIG.6

START

SET IMAGING MENU — 100

BREAST IN CONTACT? — 102
N / Y

START COMPRESSION PLATE MOVEMENT — 104

IMAGING CONDITION SETTING PROCESSING (PRE-IRRADIATION) — 106

RADIOGRAPHIC IMAGING PROCESSING — 108

OUTPUT RADIOGRAPHIC IMAGE DATA — 110

END

# FIG.7

```
┌──────────────────────────────┐
│  IMAGING CONDITION SETTING   │
│      PROCESSING START        │
└──────────────────────────────┘
              │
              ▼
         ◇ COMPRESSION          202
    N ◇  PLATE STOPPED? ◇
              │ Y
              ▼
┌──────────────────────────────┐ 204
│     ACQUIRE COMPRESSION      │
│     PLATE POSITION DATA      │
└──────────────────────────────┘
              │
              ▼
┌──────────────────────────────┐ 206
│  ACQUIRE ANGLE OF INCLINATION│
│     OF COMPRESSION PLATE     │
└──────────────────────────────┘
              │
              ▼
┌──────────────────────────────────┐ 208
│ COMPUTE SEPARATION t CORRESPONDING│
│        TO EACH AEC SENSOR         │
└──────────────────────────────────┘
              │
              ▼
┌──────────────────────────────┐ 210
│  COMPUTE CORRECTION COEFFICIENT│
│      FOR EACH AEC SENSOR     │
└──────────────────────────────┘
              │
              ▼
┌──────────────────────────────┐ 212
│      STORE CORRECTION        │
│        COEFFICIENTS          │
└──────────────────────────────┘
              │
              ▼
┌──────────────────────────────┐ 214
│    PERFORM PRE-IRRADIATION   │
└──────────────────────────────┘
              │
              ▼
┌──────────────────────────────┐ 216
│   ACQUIRE MEASUREMENT VALUE  │
│      FOR EACH AEC SENSOR     │
└──────────────────────────────┘
              │
              ▼
┌──────────────────────────────┐ 218
│   CORRECT MEASUREMENT VALUES │
│ USING CORRECTION COEFFICIENTS│
└──────────────────────────────┘
              │
              ▼
┌──────────────────────────────┐ 220
│    DETERMINE AND INSTRUCT    │
│   IMAGING CONDITIONS BASED   │
│      ON CORRECTED VALUES     │
└──────────────────────────────┘
              │
              ▼
         ┌─────────┐
         │   END   │
         └─────────┘
```

FIG.8

EP 2 649 942 A1

## FIG.9

# FIG.10

```
      ┌─────────────────────────────┐
      │  START RADIOGRAPHIC IMAGE    │
      │   CORRECTION PROCESSING      │
      └─────────────────────────────┘
                    │
      ┌─────────────────────────────┐  300
      │     ACQUIRE  IMAGE  DATA     │
      └─────────────────────────────┘
                    │
      ┌─────────────────────────────┐  302
      │      READ  CORRECTION        │
      │       COEFFICIENTS           │
      └─────────────────────────────┘
                    │
      ┌─────────────────────────────┐  304
      │  CORRECT  IMAGE  DATA  USING │
      │   CORRECTION  COEFFICIENTS   │
      └─────────────────────────────┘
                    │
      ┌─────────────────────────────┐
      │             END             │
      └─────────────────────────────┘
```

FIG.11

EP 2 649 942 A1

FIG.12

RADIOGRAPHIC IMAGING DEVICE

IMAGING DEVICE CONTROLLER

PROGRAM

COMMUNICATION
I/F SECTION

RADIATION IRRADIATION
SECTION

RADIATION SOURCE

RADIATION DETECTOR

PIXEL        AEC
             PIXEL

ANGLE
SENSOR

OPERATION
PANEL

STORAGE
SECTION

EP 2 649 942 A1

## FIG.13

START IMAGING CONDITION
SETTING PROCESSING

COMPRESSION
PLATE STOPPED?

ACQUIRE COMPRESSION PLATE POSITION DATA

ACQUIRE ANGLE OF INCLINATION OF
COMPRESSION PLATE

READ SEPARATIONS $t_1$, $t_2$ FROM
STORAGE SECTION

COMPUTE y1, y2 CORRESPONDING TO
SEPARATIONS t1, t2, DETERMINE
AND STORE FIRST TO THIRD REGIONS

PERFORM PRE-IRRADIATION

ACQUIRE DETECTION VALUES FOR EACH
REGION AT SPECIFIC AEC PIXEL
FREQUENCY (DENSITY)

READ CORRECTION COEFFICIENTS FOR
EACH REGION, CORRECT DETECTION
VALUES FOR EACH REGION USING
CORRECTION COEFFICIENTS

DETERMINE/INSTRUCT IMAGING CONDITIONS
BASED ON CORRECTED VALUES

END

# FIG.14

# FIG.15

```
     ┌─────────────────────────────┐
     │  START RADIOGRAPHIC IMAGE   │
     │    CORRECTION PROCESSING    │
     └─────────────────────────────┘
                    │
     ┌─────────────────────────────┐
     │      ACQUIRE IMAGE DATA      │
     └─────────────────────────────┘
                    │
     ┌─────────────────────────────┐
     │    READ DATA FOR EACH REGION │
     └─────────────────────────────┘
                    │
  ┌────────────────────────────────────┐
  │  CORRECT IMAGE DATA FOR EACH REGION │
  │   USING CORRECTION COEFFICIENTS    │
  └────────────────────────────────────┘
                    │
     ┌─────────────────────────────┐
     │             END             │
     └─────────────────────────────┘
```

# FIG.16

```
┌─────────────────────────────────┐
│     START IMAGING CONDITION      │
│        SETTING PROCESSING        │
└─────────────────────────────────┘
                 │
      ┌──────────▼─────────┐
      │      ◇ COMPRESSION ◇
      └─────◇ PLATE STOPPED? ◇
                 │
┌────────────────▼────────────────┐
│     ACQUIRE COMPRESSION PLATE    │
│          POSITION DATA           │
└────────────────┬────────────────┘
┌────────────────▼────────────────┐
│    ACQUIRE ANGLE OF INCLINATION OF │
│          COMPRESSION PLATE       │
└────────────────┬────────────────┘
┌────────────────▼────────────────┐
│ READ SEPARATIONS t1, t2 FROM STORAGE SECTION │
└────────────────┬────────────────┘
┌────────────────▼────────────────┐
│     COMPUTE y1, y2 CORRESPONDING TO │
│      SEPARATIONS t1, t2, DETERMINE │
│     AND STORE FIRST TO THIRD REGIONS │
└────────────────┬────────────────┘
┌────────────────▼────────────────┐
│       PERFORM PRE-IRRADIATION    │
└────────────────┬────────────────┘
┌────────────────▼────────────────┐
│   ACQUIRE DETECTION VALUES FROM SPECIFIC │
│ NUMBER OF AEC PIXELS AT SPECIFIC POSITIONS │
│            IN EACH REGION        │
└────────────────┬────────────────┘
┌────────────────▼────────────────┐
│ COMPUTE AVERAGE VALUES OF DETECTION VALUES │
│       FOR EACH SPECIFIC POSITION │
└────────────────┬────────────────┘
┌────────────────▼────────────────┐
│   READ CORRECTION COEFFICIENTS FOR EACH │
│    REGION, AND CORRECT AVERAGE VALUES FOR │
│ EACH REGION USING CORRECTION COEFFICIENTS │
└────────────────┬────────────────┘
┌────────────────▼────────────────┐
│ DETERMINE/INSTRUCT IMAGING CONDITIONS │
│       BASED ON CORRECTED VALUES  │
└────────────────┬────────────────┘
          ┌──────▼──────┐
          │     END     │
          └─────────────┘
```

# FIG.17

## FIG.18

```
┌─────────────────────────────────┐
│      START IMAGING CONDITION     │
│        SETTING PROCESSING        │
└─────────────────────────────────┘
                  │
                  ▼
         ╱────────────────╲
        ╱   COMPRESSION     ╲
        ╲  PLATE STOPPED?   ╱
         ╲────────────────╱
                  │
                  ▼
┌─────────────────────────────────┐
│     ACQUIRE COMPRESSION PLATE    │
│          POSITION DATA           │
└─────────────────────────────────┘
                  │
                  ▼
┌─────────────────────────────────┐
│    ACQUIRE ANGLE OF INCLINATION OF│
│         COMPRESSION PLATE        │
└─────────────────────────────────┘
                  │
                  ▼
┌─────────────────────────────────┐
│ READ SEPARATIONS t1, t2 FROM STORAGE SECTION│
└─────────────────────────────────┘
                  │
                  ▼
┌─────────────────────────────────┐
│     COMPUTE y1, y2 CORRESPONDING TO│
│      SEPARATIONS t1, t2, DETERMINE│
│     AND STORE FIRST TO THIRD REGIONS│
└─────────────────────────────────┘
                  │
                  ▼
┌─────────────────────────────────┐
│         PERFORM PRE-IRRADIATION  │
└─────────────────────────────────┘
                  │
                  ▼
┌─────────────────────────────────┐
│  ACQUIRE DETECTION VALUES FROM PREDETERMINED│
│   NUMBER ($k_x \times k_y$) OF AEC PIXELS (DETECTION│
│   SENSOR) CENTERING AROUND EACH SPECIFIC│
│        POSITION FOR EACH REGION  │
└─────────────────────────────────┘
                  │
                  ▼
┌─────────────────────────────────┐
│      COMPUTE AVERAGE VALUE FOR EACH│
│           DETECTION SENSOR       │
└─────────────────────────────────┘
                  │
                  ▼
┌─────────────────────────────────┐
│ READ CORRECTION COEFFICIENT FOR EACH REGION,│
│ AND CORRECT AVERAGE VALUES FOR EACH REGION│
│      USING CORRECTION COEFFICIENTS│
└─────────────────────────────────┘
                  │
                  ▼
┌─────────────────────────────────┐
│ DETERMINE AND INSTRUCT IMAGING CONDITIONS│
│      BASED ON CORRECTED VALUES   │
└─────────────────────────────────┘
                  │
                  ▼
         ┌─────────────┐
         │     END     │
         └─────────────┘
```

## FIG.19

## FIG.20

```
      ┌─────────────────────────────┐
      │   START IMAGING CONDITION    │
      │     SETTING PROCESSING       │
      └─────────────────────────────┘
                    │
        ┌───────────┤
        │           ▼
        │     ╱────────────╲
        │    ╱ COMPRESSION   ╲
        └───▷  PLATE STOPPED? ╲
             ╲              ╱
              ╲────────────╱
                    │
      ┌─────────────────────────────┐
      │   ACQUIRE COMPRESSION PLATE  │
      │       POSITION DATA          │
      └─────────────────────────────┘
                    │
      ┌─────────────────────────────┐
      │  ACQUIRE ANGLE OF INCLINATION OF │
      │       COMPRESSION PLATE      │
      └─────────────────────────────┘
                    │
      ┌─────────────────────────────┐
      │ READ SEPARATIONS t1, t2 FROM STORAGE SECTION │
      └─────────────────────────────┘
                    │
      ┌─────────────────────────────┐
      │   COMPUTE y1, y2 CORRESPONDING TO │
      │     SEPARATIONS t1, t2, DETERMINE │
      │   AND STORE FIRST TO THIRD REGIONS │
      └─────────────────────────────┘
                    │
      ┌─────────────────────────────┐
      │     PERFORM PRE-IRRADIATION  │
      └─────────────────────────────┘
                    │
      ┌─────────────────────────────┐
      │      GENERATE PRE-IMAGE      │
      └─────────────────────────────┘
                    │
      ┌─────────────────────────────┐
      │      DETECT BREAST IMAGE     │
      └─────────────────────────────┘
                    │
      ┌─────────────────────────────┐
      │ ACQUIRE DETECTION VALUES FROM SPECIFIC │
      │ FREQUENCY (DENSITY) OF AEC PIXELS FROM WITHIN │
      │   BREAST IMAGE FOR EACH REGION │
      └─────────────────────────────┘
                    │
      ┌─────────────────────────────┐
      │ READ CORRECTION COEFFICIENT FOR EACH REGION, │
      │ AND CORRECT DETECTION VALUES FOR EACH REGION │
      │    USING CORRECTION COEFFICIENTS │
      └─────────────────────────────┘
                    │
      ┌─────────────────────────────┐
      │ DETERMINE AND INSTRUCT IMAGING CONDITIONS │
      │      BASED ON CORRECTED VALUES │
      └─────────────────────────────┘
                    │
      ┌─────────────────────────────┐
      │             END              │
      └─────────────────────────────┘
```

## FIG.21

FIG.22

RADIOGRAPHIC IMAGING DEVICE

IMAGING DEVICE CONTROLLER

PROGRAM

COMMUNICATION I/F SECTION

RADIATION IRRADIATION SECTION

RADIATION SOURCE

RADIATION DETECTOR

PIXEL (AEC PIXEL)

ANGLE SENSOR

OPERATION PANEL

STORAGE SECTION

EP 2 649 942 A1

## FIG.23

```
┌─────────────────────────────┐
│   START IMAGING CONDITION   │
│     SETTING PROCESSING      │
└─────────────────────────────┘
              │
              ▼
         ╱─────────╲
        ╱ COMPRESSION ╲───┐
        ╲ PLATE STOPPED? ╱
         ╲─────────╱
              │
              ▼
┌─────────────────────────────┐
│   ACQUIRE COMPRESSION PLATE  │
│        POSITION DATA         │
└─────────────────────────────┘
              │
              ▼
┌─────────────────────────────┐
│  ACQUIRE ANGLE OF INCLINATION OF │
│      COMPRESSION PLATE       │
└─────────────────────────────┘
              │
              ▼
┌─────────────────────────────┐
│ READ SEPARATIONS t1, t2 FROM STORAGE SECTION │
└─────────────────────────────┘
              │
              ▼
┌─────────────────────────────┐
│  COMPUTE y1, y2 CORRESPONDING TO │
│   SEPARATIONS t1, t2, DETERMINE  │
│  AND STORE FIRST TO THIRD REGIONS │
└─────────────────────────────┘
              │
              ▼
┌─────────────────────────────┐
│     PERFORM PRE-IRRADIATION  │
└─────────────────────────────┘
              │
              ▼
┌─────────────────────────────┐
│ ACQUIRE DETECTION VALUES FROM PREDETERMINED │
│  NUMBER (kx × ky) OF AEC PIXELS FOR EACH │
│  DETECTION SENSOR FOR EACH REGION │
└─────────────────────────────┘
              │
              ▼
┌─────────────────────────────┐
│    COMPUTE AVERAGE VALUE FOR EACH │
│       DETECTION SENSOR       │
└─────────────────────────────┘
              │
              ▼
┌─────────────────────────────┐
│ READ CORRECTION COEFFICIENT FOR EACH REGION, │
│ AND CORRECT AVERAGE VALUES FOR EACH REGION │
│   USING CORRECTION COEFFICIENTS │
└─────────────────────────────┘
              │
              ▼
┌─────────────────────────────┐
│ DETERMINE AND INSTRUCT IMAGING CONDITIONS │
│     BASED ON CORRECTED VALUES │
└─────────────────────────────┘
              │
              ▼
┌─────────────────────────────┐
│             END             │
└─────────────────────────────┘
```

# FIG.24

Europäisches
Patentamt
European
Patent Office
Office européen
des brevets

# EUROPEAN SEARCH REPORT

Application Number

EP 13 16 2335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/181360 A1 (HEMMENDORFF MAGNUS [SE]) 31 July 2008 (2008-07-31)<br>* abstract *<br>* figure 2 *<br>* paragraph [0011] - paragraph [0032] *<br>----- | 1-20 | INV.<br>A61B6/04<br>A61B6/00 |
| A | US 5 506 877 A (NIKLASON LOREN T [US] ET AL) 9 April 1996 (1996-04-09)<br>* abstract *<br>* figure 3 *<br>* column 5, line 25 - column 8, line 64 *<br>----- | 14,19 | |

TECHNICAL FIELDS
SEARCHED (IPC)

A61B

The present search report has been drawn up for all claims

| Place of search | Date of completion of the search | Examiner |
|---|---|---|
| The Hague | 26 June 2013 | Moehrs, Sascha |

EPO FORM 1503 03.82 (P04C01)

**ANNEX TO THE EUROPEAN SEARCH REPORT
ON EUROPEAN PATENT APPLICATION NO.**

EP 13 16 2335

This annex lists the patent family members relating to the patent documents cited in the above-mentioned European search report.
The members are as contained in the European Patent Office EDP file on
The European Patent Office is in no way liable for these particulars which are merely given for the purpose of information.

26-06-2013

| Patent document cited in search report | Publication date | Patent family member(s) | Publication date |
|---|---|---|---|
| US 2008181360 A1 | 31-07-2008 | NONE | |
| US 5506877 A | 09-04-1996 | NONE | |

**EP 2 649 942 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005509482 A
- JP 2008086383 A